(19) Europäisches Patentamt
European Patent Office
Office européen des brevets

(11) **EP 4 230 656 A1**

(12) **EUROPEAN PATENT APPLICATION**
published in accordance with Art. 153(4) EPC

(43) Date of publication:
**23.08.2023 Bulletin 2023/34**

(21) Application number: **21879467.5**

(22) Date of filing: **14.10.2021**

(51) International Patent Classification (IPC):
*C07K 16/30* (2006.01)   *A61K 39/395* (2006.01)
*A61P 35/00* (2006.01)

(52) Cooperative Patent Classification (CPC):
**A61K 39/395; A61P 35/00; C07K 16/30**

(86) International application number:
**PCT/CN2021/123731**

(87) International publication number:
**WO 2022/078424 (21.04.2022 Gazette 2022/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.10.2020 CN 202011099107**

(71) Applicants:
• **Shanghai Hansoh Biomedical Co., Ltd.**
**Shanghai 201203 (CN)**
• **Jiangsu Hansoh Pharmaceutical Group Co., Ltd.**
**Lianyungang, Jiangsu 222047 (CN)**

(72) Inventors:
• **MAO, Dongjie**
**Shanghai 201203 (CN)**
• **LUO, Yan**
**Shanghai 201203 (CN)**
• **XIE, Yuejun**
**Shanghai 201203 (CN)**

(74) Representative: **EP&C**
**P.O. Box 3241**
**2280 GE Rijswijk (NL)**

Remarks:
The complete document including Reference
Table(s) and the Sequence Listing(s) can be
downloaded from the EPO website

(54) **ANTI-TROP-2 ANTIBODY, ANTIGEN-BINDING FRAGMENT THEREOF OR MUTANT THEREOF, AND MEDICAL USE THEREOF**

(57) The present invention relates to an anti-TROP-2 antibody, an antigen-binding fragment thereof or a mutant thereof, and a medical use thereof. Furthermore, the present application relates to a humanized anti-TROP-2 antibody mutant, a pharmaceutical composition comprising an anti-TROP-2 antibody mutant, a use thereof in the preparation of a drug for treating TROP-2-mediated diseases or conditions, and a use in tumor detection and diagnosis.

EP 4 230 656 A1

**Description**

The present application claims the priority of the patent application No. 202011099107.8 filed on October 14, 2020.

**FIELD OF THE INVENTION**

The present disclosure relates to an anti-TROP-2 antibody, an antigen-binding fragment thereof or a mutant thereof, relates to a chimeric antibody or a humanized antibody comprising the CDR region of the anti-TROP-2 antibody or a mutant thereof, and relates to a pharmaceutical composition comprising human anti-TROP-2 antibody, an antigen-binding fragment thereof or a mutant thereof, as well as relates to the use thereof as an anti-cancer drug and a use thereof for the detection or diagnosis of tumors.

**BACKGROUND OF THE INVENTION**

With the deepening of the study of tumor genomics, proteomics and signaling pathways, the interaction of oncogenes and tumor suppressor genes in tumor cells and their impact on the tumor microenvironment have become clearer, which also makes it possible to design new anti-tumor therapeutic schemes for specific molecular targets of tumors.

Molecular targeted treatment for tumors is a new treatment mode different from traditional surgery, radiotherapy and chemotherapy, and the advantage is that the drug usually only binds to the corresponding target, and kills or inhibits the target cell by directly affecting the function of the target molecule or by the physical or chemical effector molecule it carries. Due to the specific target, the drug is usually highly selective, and can effectively kill or inhibit the target cell while producing no or only little toxic side effect on normal tissue cells. Therefore, the development of molecular targeted drugs has become a hot spot in tumor clinical research.

Trophoblast cell surface antigen 2 (TROP-2), also known as tumor-associated calcium signal transducer 2 (TACSTD2), is a cellular transmembrane glycoprotein encoded by the *TACSTD2* gene. TROP-2 consists of about 323 amino acids, of which the signal peptide comprises 26 amino acids, the extracellular region comprises 248 amino acids, the transmembrane region comprises 23 amino acids, and the cytosolic region comprises 26 amino acids. There are 4 heterogeneous N-linked glycosylation sites in the extracellular domain of TROP-2, and the apparent molecular weight increases by 11 to 13 KD after adding sugar chains. In the TACSTD gene family, the extracellular domain has a characteristic thyroglobulin (TY) sequence, which is usually thought to be associated with the proliferation, infiltration and metastasis of cancer cells.

To date, no physiologic ligand of TROP-2 has been identified, and the molecular function of TROP-2 has not been elucidated. However, due to the phosphorylation at the intracellular Serine 303 residue by protein kinase C (PKC, which belongs to $Ca^{2+}$-dependent protein kinases) and the presence of PIP2-binding sequence in the intracellular domain, TROP-2 is demonstrated to have signaling functions in tumor cells.

A large number of clinical studies and literature reports have shown that TROP-2 antigen is overexpressed in a variety of epithelial-derived cancers, such as gastric cancer, lung cancer, colorectal cancer, ovarian cancer, breast cancer, prostate cancer, pancreatic cancer, liver cancer, esophageal cancer and the like. The TROP-2 antigen is rarely expressed or not expressed in normal tissues in adults, only rarely expressed in cells in the epithelial region, and the expression level is also lower than that in cancer cells, indicating that TROP-2 is associated with tumor formation. Overexpression of TROP-2 in tumor tissues is closely related to poor prognosis and metastasis of cancer cells, while affecting the overall survival of subjects. As a result, TROP-2 has become a compelling target in molecular targeted treatments for tumors.

Currently, studies have reported the anti-tumor effects of a variety of TROP-2 antibodies:

U.S. Patent No. 5840854 reports the cytotoxicity of a cytotoxin-conjugated anti-TROP-2 monoclonal antibody (BR110) against human cancer cell lines H3619, H2987, MCF-7, H3396 and H2981.

U.S. Patent No. 6653104 discloses an antibody (RS7), which was labeled with radioactive substances and tested in an *in vivo* model, and showed antitumor activity in a nude mice xenograft model, but no antitumor effect of the naked antibody was reported.

In addition, U.S. Patent No. 7420040 reports that isolated monoclonal antibodies, generated by hybridoma cell lines AR47A6.4.2 or AR52A301.5 obtained by immunizing mice with human ovarian cancer tissue, bind to TROP-2 antigens and display antitumor activity in a nude mice xenograft model.

CN102827282A discloses a genetically engineered human anti-TROP-2 antibody IgG and the use thereof. The results of *in vitro* tests showed that this anti-TROP-2 antibody IgG had a significant inhibitory effect on the proliferation of pancreatic cancer cells.

CN104114580A discloses an antibody (especially a humanized antibody) that specifically reacts with TROP-2 antigen and has antitumor activity *in vivo,* as well as a hybridoma that produces the antibody, a complex of the antibody and an agent, a pharmaceutical composition for the diagnosis or treatment of tumors, a detection method for tumors, and a kit for the detection or diagnosis of tumors.

However, it is challenging to find monoclonal antibodies with high affinity, high specificity, and potent cytotoxicity or tumor-killing/inhibitory/degenerative activity. Therefore, there is still an urgent need to develop TROP-2 antibodies and other immunotherapeutic agents with good efficacy, high safety and suitable for human subjects.

## SUMMARY OF THE INVENTION

The present disclosure provides an anti-TROP-2 antibody, an antigen-binding fragment thereof or a mutant thereof, which comprises a heavy chain variable region and a light chain variable region.

In a preferred embodiment of the anti-TROP-2 antibody, antigen-binding fragment thereof or mutant thereof of the present disclosure, wherein:

the heavy chain variable region comprises at least one HCDR as shown in the sequence selected from the group consisting of:

SEQ ID NO: 4, SEQ ID NO: 43 and SEQ ID NO: 6;
and
the light chain variable region comprises at least one LCDR as shown in the sequence selected from the group consisting of:
SEQ ID NO: 7, SEQ ID NO: 8 and SEQ ID NO: 9;
wherein, SEQ ID NO: 43 is as shown in RIDPXDSETHYNQKFKD, the X is selected from the group consisting of amino acid residues R, Y, Q, L, T, I, F, E and A.

In a preferred embodiment of the present disclosure, the heavy chain variable region HCDR2 is selected from the group consisting of SEQ ID NO: 26, SEQ ID NO: 27, SEQ ID NO: 28, SEQ ID NO: 29, SEQ ID NO: 30, SEQ ID NO: 39, SEQ ID NO: 40, SEQ ID NO: 41 and SEQ ID NO: 42.

In a preferred embodiment of the anti-TROP-2 antibody, antigen-binding fragment thereof or mutant thereof of the present disclosure, the antibody heavy chain variable region comprises:

HCDR1 as shown in SEQ ID NO: 4, HCDR2 as shown in SEQ ID NO: 26 and HCDR3 as shown in SEQ ID NO: 6; or
HCDR1 as shown in SEQ ID NO: 4, HCDR2 as shown in SEQ ID NO: 27 and HCDR3 as shown in SEQ ID NO: 6; or
HCDR1 as shown in SEQ ID NO: 4, HCDR2 as shown in SEQ ID NO: 28 and HCDR3 as shown in SEQ ID NO: 6; or
HCDR1 as shown in SEQ ID NO: 4, HCDR2 as shown in SEQ ID NO: 29 and HCDR3 as shown in SEQ ID NO: 6; or
HCDR1 as shown in SEQ ID NO: 4, HCDR2 as shown in SEQ ID NO: 30 and HCDR3 as shown in SEQ ID NO: 6; or
HCDR1 as shown in SEQ ID NO: 4, HCDR2 as shown in SEQ ID NO: 39 and HCDR3 as shown in SEQ ID NO: 6; or
HCDR1 as shown in SEQ ID NO: 4, HCDR2 as shown in SEQ ID NO: 40 and HCDR3 as shown in SEQ ID NO: 6; or
HCDR1 as shown in SEQ ID NO: 4, HCDR2 as shown in SEQ ID NO: 41 and HCDR3 as shown in SEQ ID NO: 6; or
HCDR1 as shown in SEQ ID NO: 4, HCDR2 as shown in SEQ ID NO: 42 and HCDR3 as shown in SEQ ID NO: 6.

In a preferred embodiment of the anti-TROP-2 antibody, antigen-binding fragment thereof or mutant thereof of the present disclosure, the light chain variable region comprises:
LCDR1 as shown in SEQ ID NO: 7, LCDR2 as shown in SEQ ID NO: 8 and LCDR3 as shown in SEQ ID NO: 9.

In a preferred embodiment of the anti-TROP-2 antibody, antigen-binding fragment thereof or mutant thereof of the present disclosure, wherein:

the heavy chain variable region comprises: HCDR1 as shown in SEQ ID NO: 4, HCDR2 as shown in SEQ ID NO: 26 and HCDR3 as shown in SEQ ID NO: 6; and
the light chain variable region comprises: LCDR1 as shown in SEQ ID NO: 7, LCDR2 as shown in SEQ ID NO: 8 and LCDR3 as shown in SEQ ID NO: 9.

In a preferred embodiment of the anti-TROP-2 antibody, antigen-binding fragment thereof or mutant thereof of the present disclosure, wherein:

the heavy chain variable region comprises: HCDR1 as shown in SEQ ID NO: 4, HCDR2 as shown in SEQ ID NO:

27 and HCDR3 as shown in SEQ ID NO: 6; and
the light chain variable region comprises: LCDR1 as shown in SEQ ID NO: 7, LCDR2 as shown in SEQ ID NO: 8 and LCDR3 as shown in SEQ ID NO: 9.

In a preferred embodiment of the anti-TROP-2 antibody, antigen-binding fragment thereof or mutant thereof of the present disclosure, wherein:

the heavy chain variable region comprises: HCDR1 as shown in SEQ ID NO: 4, HCDR2 as shown in SEQ ID NO: 28 and HCDR3 as shown in SEQ ID NO: 6; and
the light chain variable region comprises: LCDR1 as shown in SEQ ID NO: 7, LCDR2 as shown in SEQ ID NO: 8 and LCDR3 as shown in SEQ ID NO: 9.

In a preferred embodiment of the anti-TROP-2 antibody, antigen-binding fragment thereof or mutant thereof of the present disclosure, wherein:

the heavy chain variable region comprises: HCDR1 as shown in SEQ ID NO: 4, HCDR2 as shown in SEQ ID NO: 29 and HCDR3 as shown in SEQ ID NO: 6; and
the light chain variable region comprises: LCDR1 as shown in SEQ ID NO: 7, LCDR2 as shown in SEQ ID NO: 8 and LCDR3 as shown in SEQ ID NO: 9.

In a preferred embodiment of the anti-TROP-2 antibody, antigen-binding fragment thereof or mutant thereof of the present disclosure, wherein:

the heavy chain variable region comprises: HCDR1 as shown in SEQ ID NO: 4, HCDR2 as shown in SEQ ID NO: 30 and HCDR3 as shown in SEQ ID NO: 6; and
the light chain variable region comprises: LCDR1 as shown in SEQ ID NO: 7, LCDR2 as shown in SEQ ID NO: 8 and LCDR3 as shown in SEQ ID NO: 9.

In a preferred embodiment of the anti-TROP-2 antibody, antigen-binding fragment thereof or mutant thereof of the present disclosure, wherein:

the heavy chain variable region comprises: HCDR1 as shown in SEQ ID NO: 4, HCDR2 as shown in SEQ ID NO: 39 and HCDR3 as shown in SEQ ID NO: 6; and
the light chain variable region comprises: LCDR1 as shown in SEQ ID NO: 7, LCDR2 as shown in SEQ ID NO: 8 and LCDR3 as shown in SEQ ID NO: 9.

In a preferred embodiment of the anti-TROP-2 antibody, antigen-binding fragment thereof or mutant thereof of the present disclosure, wherein:

the heavy chain variable region comprises: HCDR1 as shown in SEQ ID NO: 4, HCDR2 as shown in SEQ ID NO: 40 and HCDR3 as shown in SEQ ID NO: 6; and
the light chain variable region comprises: LCDR1 as shown in SEQ ID NO: 7, LCDR2 as shown in SEQ ID NO: 8 and LCDR3 as shown in SEQ ID NO: 9.

In a preferred embodiment of the anti-TROP-2 antibody, antigen-binding fragment thereof or mutant thereof of the present disclosure, wherein:

the heavy chain variable region comprises: HCDR1 as shown in SEQ ID NO: 4, HCDR2 as shown in SEQ ID NO: 41 and HCDR3 as shown in SEQ ID NO: 6; and
the light chain variable region comprises: LCDR1 as shown in SEQ ID NO: 7, LCDR2 as shown in SEQ ID NO: 8 and LCDR3 as shown in SEQ ID NO: 9.

In a preferred embodiment of the anti-TROP-2 antibody, antigen-binding fragment thereof or mutant thereof of the present disclosure, wherein:

the heavy chain variable region comprises: HCDR1 as shown in SEQ ID NO: 4, HCDR2 as shown in SEQ ID NO: 42 and HCDR3 as shown in SEQ ID NO: 6; and
the light chain variable region comprises: LCDR1 as shown in SEQ ID NO: 7, LCDR2 as shown in SEQ ID NO: 8

and LCDR3 as shown in SEQ ID NO: 9.

In a preferred embodiment of the anti-TROP-2 antibody, antigen-binding fragment thereof or mutant thereof of the present disclosure, the antibody is a murine antibody, a chimeric antibody, a human antibody or a humanized antibody.

In a preferred embodiment of the anti-TROP-2 antibody, antigen-binding fragment thereof or mutant thereof of the present disclosure, the anti-TROP-2 antibody or antigen-binding fragment thereof further comprises a constant region derived from human IgG1, IgG2, IgG3 or IgG4 or a mutant thereof.

In a further preferred embodiment of the present disclosure, the anti-TROP-2 antibody, antigen-binding fragment thereof or mutant thereof further comprises a heavy chain constant region derived from human IgG1, IgG2 or IgG4 or a mutant thereof.

In a further preferred embodiment of the present disclosure, the anti-TROP-2 antibody, antigen-binding fragment thereof or mutant thereof further comprises a heavy chain constant region derived from IgG1 with enhanced ADCC toxicity after amino acid mutation.

In a further preferred embodiment of the present disclosure, the anti-TROP-2 antibody, antigen-binding fragment thereof or mutant thereof further comprises a heavy chain constant region of IgG1 introduced with 239D and 241L mutations.

In a further preferred embodiment of the present disclosure, the anti-TROP-2 antibody, antigen-binding fragment thereof or mutant thereof further comprises a heavy chain constant region selected from SEQ ID NO: 12.

In a further preferred embodiment of the present disclosure, the anti-TROP-2 antibody, antigen-binding fragment thereof or mutant thereof further comprises a light chain constant region derived from human κ chain, λ chain or a mutant thereof.

In a further preferred embodiment of the present disclosure, the anti-TROP-2 antibody, antigen-binding fragment thereof or mutant thereof further comprises a light chain constant region selected from SEQ ID NO: 13.

In a preferred embodiment of the anti-TROP-2 antibody, antigen-binding fragment thereof or mutant thereof of the present disclosure, the anti-TROP-2 antibody, antigen-binding fragment thereof or mutant thereof comprises a heavy chain variable region selected from the group consisting of SEQ ID NO: 16, SEQ ID NO: 17, SEQ ID NO: 18, SEQ ID NO: 19, SEQ ID NO: 20, SEQ ID NO: 31, SEQ ID NO: 32, SEQ ID NO: 33, SEQ ID NO: 34, or a heavy chain variable region having at least 70%, 75%, 80%, 85%, 90%, 95% or 99% identity with the same.

In a preferred embodiment of the anti-TROP-2 antibody, antigen-binding fragment thereof or mutant thereof of the present disclosure, the anti-TROP-2 antibody, antigen-binding fragment thereof or mutant thereof comprises the light chain variable region of SEQ ID NO: 11, or a light chain variable region having at least 70%, 75%, 80%, 85%, 90%, 95% or 99% identity with the same.

In a preferred embodiment of the present disclosure, the anti-TROP-2 antibody or mutant thereof comprises a heavy chain selected from the group consisting of SEQ ID NO: 21, SEQ ID NO: 22, SEQ ID NO: 23, SEQ ID NO: 24, SEQ ID NO: 25, SEQ ID NO: 35, SEQ ID NO: 36, SEQ ID NO: 37, SEQ ID NO: 38, or a full-length heavy chain having at least 80%, 85%, 90%, 95% or 99% identity with the same.

In a preferred embodiment of the present disclosure, the anti-TROP-2 antibody or mutant thereof comprises the light chain of SEQ ID NO: 15, or a full-length light chain having at least 80%, 85%, 90%, 95% or 99% identity with the same.

In a preferred embodiment of the anti-TROP-2 antibody, antigen-binding fragment thereof or mutant thereof of the present disclosure, wherein:

the anti-TROP-2 antibody, antigen-binding fragment thereof or mutant thereof comprises the heavy chain variable region as shown in SEQ ID NO: 16, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or,

the anti-TROP-2 antibody, antigen-binding fragment thereof or mutant thereof comprises the heavy chain variable region as shown in SEQ ID NO: 17, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or,

the anti-TROP-2 antibody, antigen-binding fragment thereof or mutant thereof comprises the heavy chain variable region as shown in SEQ ID NO: 18, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or,

the anti-TROP-2 antibody, antigen-binding fragment thereof or mutant thereof comprises the heavy chain variable region as shown in SEQ ID NO: 19, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or,

the anti-TROP-2 antibody, antigen-binding fragment thereof or mutant thereof comprises the heavy chain variable region as shown in SEQ ID NO: 20, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or,

the anti-TROP-2 antibody, antigen-binding fragment thereof or mutant thereof comprises the heavy chain variable region as shown in SEQ ID NO: 31, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or,

the anti-TROP-2 antibody, antigen-binding fragment thereof or mutant thereof comprises the heavy chain variable region as shown in SEQ ID NO: 32, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or,

the anti-TROP-2 antibody, antigen-binding fragment thereof or mutant thereof comprises the heavy chain variable region as shown in SEQ ID NO: 33, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or,

the anti-TROP-2 antibody, antigen-binding fragment thereof or mutant thereof comprises the heavy chain variable region as shown in SEQ ID NO: 34, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same.

In a preferred embodiment of the anti-TROP-2 antibody, antigen-binding fragment thereof or mutant thereof of the present disclosure, wherein:

the anti-TROP-2 antibody or mutant thereof comprises the heavy chain as shown in SEQ ID NO: 21, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or,

the anti-TROP-2 antibody or mutant thereof comprises the heavy chain as shown in SEQ ID NO: 22, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or,

the anti-TROP-2 antibody or mutant thereof comprises the heavy chain as shown in SEQ ID NO: 23, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or,

the anti-TROP-2 antibody or mutant thereof comprises the heavy chain as shown in SEQ ID NO: 24, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or,

the anti-TROP-2 antibody or mutant thereof comprises the heavy chain as shown in SEQ ID NO: 25, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or,

the anti-TROP-2 antibody or mutant thereof comprises the heavy chain as shown in SEQ ID NO: 35, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or,

the anti-TROP-2 antibody or mutant thereof comprises the heavy chain as shown in SEQ ID NO: 36, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or,

the anti-TROP-2 antibody or mutant thereof comprises the heavy chain as shown in SEQ ID NO: 37, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or,

the anti-TROP-2 antibody or mutant thereof comprises the heavy chain as shown in SEQ ID NO: 38, or a heavy chain having at least 80%, 85%, 90%, 95% or 99% identity with the same, and the light chain as shown in SEQ ID NO: 15, or a light chain having at least 80%, 85%, 90%, 95% or 99% identity with the same.

The present disclosure also relates to a polynucleotide encoding the above-mentioned anti-TROP-2 antibody, antigen-binding fragment thereof or mutant thereof.

The present disclosure also relates to an expression vector comprising the above-mentioned polynucleotide.

The present disclosure also relates to a host cell transformed with the above-mentioned expression vector.

The present disclosure also relates to a preferred embodiment, in which the host cell is selected from the group consisting of bacterium, yeast and mammalian cell.

The present disclosure also relates to a further preferred embodiment, in which the host cell is selected from the group consisting of *Escherichia coli, Pichia pastoris,* CHO cell and HEK293 cell.

The present disclosure also relates to a method of producing the above-mentioned anti-TROP-2 antibody, antigen-binding fragment thereof or mutant thereof, the method comprises the following steps: culturing the above-mentioned host cell, preferably HEK293 cells; isolating the antibody from the culture, preferably isolating the antibody from the cell culture fluid; and purifying the antibody, preferably purifying the antibody by chromatographic methods.

The present disclosure also relates to a pharmaceutical composition comprising the above-mentioned anti-TROP-2 antibody, antigen-binding fragment thereof or mutant thereof, as well as a pharmaceutically acceptable excipient, diluent or carrier.

The present disclosure also relates to a detection or diagnostic kit comprising the above-mentioned anti-TROP-2 antibody, antigen-binding fragment thereof or mutant thereof.

The present disclosure also relates to use of the above-mentioned anti-TROP-2 antibody, antigen-binding fragment thereof or mutant thereof in the preparation of a medicament, the medicament is for treating or preventing a TROP-2-mediated disease or condition.

The present disclosure also relates to use of the above-mentioned anti-TROP-2 antibody, antigen-binding fragment thereof or mutant thereof in the preparation of a reagent, wherein the reagent is for detecting, diagnosing and prognosing a TROP-2-mediated disease or condition.

The present disclosure also relates to a preferred embodiment of the above-mentioned use, wherein the TROP-2-mediated disease or condition is a cancer; preferably, the TROP-2-mediated disease or condition is a cancer expressing TROP-2; preferably, the cancer is selected from the group consisting of breast cancer, non-small cell lung cancer, ovarian cancer, prostate cancer, pancreatic cancer, kidney cancer, lung cancer, liver cancer, gastric cancer, colon cancer, bladder cancer, esophageal cancer, cervical cancer, gallbladder cancer, glioblastoma and melanoma.

The present disclosure also relates to a preferred embodiment of the above-mentioned anti-TROP-2 antibody, antigen-binding fragment thereof or mutant thereof, which is used for treating or preventing a TROP-2-mediated disease, the disease is selected from the group consisting of breast cancer, non-small cell lung cancer, ovarian cancer, prostate cancer, pancreatic cancer, kidney cancer, lung cancer, liver cancer, gastric cancer, colon cancer, bladder cancer, esophageal cancer, cervical cancer, gallbladder cancer, glioblastoma and melanoma.

The present disclosure also relates to a preferred embodiment of the above-mentioned anti-TROP-2 antibody, antigen-binding fragment thereof or mutant thereof, which is used for detecting, diagnosing and prognosing a TROP-2-mediated disease, the disease is selected from the group consisting of breast cancer, non-small cell lung cancer, ovarian cancer, prostate cancer, pancreatic cancer, kidney cancer, lung cancer, liver cancer, gastric cancer, colon cancer, bladder cancer, esophageal cancer, cervical cancer, gallbladder cancer, glioblastoma and melanoma.

The present disclosure also relates to a method of treating or preventing a TROP-2-mediated disease, comprising the following steps: providing to a subject in need a therapeutically or prophylactically effective amount of the anti-TROP-2 antibody, antigen-binding fragment thereof or mutant thereof as described above, or providing to a subject in need a therapeutically or prophylactically effective amount of the pharmaceutical composition according to the present disclosure, wherein the TROP-2-mediated disease is selected from the group consisting of breast cancer, non-small cell lung cancer, ovarian cancer, prostate cancer, pancreatic cancer, kidney cancer, lung cancer, liver cancer, gastric cancer, colon cancer, bladder cancer, esophageal cancer, cervical cancer, gallbladder cancer, glioblastoma and melanoma.

## DETAILED DESCRIPTION OF THE INVENTION

Terms

For easier understanding of the present disclosure, certain technical and scientific terms are specifically defined below. Unless otherwise clearly defined elsewhere in this document, all other technical and scientific terms used herein have the meanings commonly understood by those of ordinary skilled in the art to which the present disclosure belongs.

The three-letter codes and one-letter codes of amino acids used in the present disclosure are as described in J. Biol. Chem, 243, p3558 (1968).

The term "antibody" described in the present disclosure refers to an immunoglobulin, which is a tetra-peptide chain structure composed of two heavy chains and two light chains linked by interchain disulfide bonds. The amino acid composition and order of arrangement of the immunoglobulin heavy chain constant regions are different, so their antigenicity is also different. Accordingly, immunoglobulins can be classified into five types, or immunoglobulin isotypes, namely IgM, IgD, IgG, IgA and IgE, and their corresponding heavy chains are $\mu$ chain, $\delta$ chain, $\gamma$ chain, $\alpha$ chain and $\varepsilon$ chain, respectively. The same type of Ig can be classified into different subclasses according to the difference in the amino acid composition of the hinge region and the number and position of heavy chain disulfide bonds. For example, IgG can be classified into IgG1, IgG2, IgG3 and IgG4. The light chain is classified into $\kappa$ chain or $\lambda$ chain according to

the difference in the constant region. Each of the five types of Ig can have a κ chain or a λ chain.

In the present disclosure, the antibody light chain can further comprise a light chain constant region, which comprises human or murine κ, λ chain or a variant thereof.

In the present disclosure, the antibody heavy chain can further comprise a heavy chain constant region, which comprises human or murine IgG1, IgG2, IgG3, IgG4 or a variant thereof.

The sequence of about 110 amino acids near the N-terminus of the antibody heavy and light chains varies greatly and is named as variable region (V region); the rest amino acid sequence near the C-terminus is relatively stable and is named as constant region (C region). The variable region comprises 3 hypervariable regions (HVRs) and 4 framework regions (FRs) with relatively conservative sequences. The 3 hypervariable regions determine the specificity of the antibody, and are also known as complementarity determining regions (CDR). Each light chain variable region (VL) and heavy chain variable region (VH) consists of 3 CDR regions and 4 FR regions, arranged from the amino terminus to the carboxyl terminus in the order of: FR1, CDR1, FR2, CDR2, FR3, CDR3, FR4. The 3 CDR regions of the light chain refer to LCDR1, LCDR2 and LCDR3; the 3 CDR regions of the heavy chain refer to HCDR1, HCDR2 and HCDR3. The number and position of the CDR amino acid residues of the VL region and VH region of the antibody or antigen-binding fragment according to the present disclosure comply with the known Kabat numbering and Kabat or ABM criteria.

The term "TROP-2" includes any naturally expressed variant or isoform of TROP-2. The antibody of the present disclosure can cross-react with TROP-2 derived from non-human species. As an alternative, the antibody can also be specific for human TROP-2 and may not show cross-reactivity with other species. TROP-2 or any variant or isoform thereof can be isolated from cells or tissues naturally expressing them, or produced by recombinant techniques using the techniques commonly used in the art and those described herein. Preferably, the anti-TROP-2 antibody targets human TROP-2 that has a normal glycosylation pattern.

The term "recombinant human antibody" includes human antibodies prepared, expressed, created or isolated by recombinant methods, and the techniques and methods involved are well known in the art, such as:

1. antibodies isolated from transgenic and transchromosomal animals (for example mice) of human immunoglobulin genes, or hybridomas prepared therefrom;
2. antibodies isolated from host cells transformed to express the antibodies, such as transfectionomas;
3. antibodies isolated from recombinant combinatorial human antibody libraries; and
4. antibodies prepared, expressed, created or isolated by techinques such as splicing human immunoglobulin gene sequences into other DNA sequences.

Such recombinant human antibodies comprise variable regions and constant regions, which utilize specific human germline immunoglobulin sequences encoded by germline genes, but also comprise subsequent rearrangements and mutations such as those occur during antibody maturation.

The term "murine antibody" in the present disclosure is a monoclonal antibody against human TROP-2 according to the knowledge and skills in the art. During preparation, the test subject is injected with TROP-2 antigen, and then hybridomas expressing antibodies with the desired sequence or functional properties are isolated. In an exemplary embodiment of the present disclosure, the murine TROP-2 antibody or antigen-binding fragment thereof may further comprise the light chain constant region of murine κ, λ chain or a variant thereof, or further comprise the heavy chain constant region of murine IgG1, IgG2, IgG3 or IgG4 or a variant thereof.

The term "human antibody" includes antibodies with variable and constant regions of human germline immunoglobulin sequences. The human antibodies of the present disclosure may include amino acid residues that are not encoded by human germline immunoglobulin sequences (such as mutations introduced by random or site-specific mutagenesis *in vitro* or by somatic mutations *in vivo*). However, the term "human antibody" does not include humanized antibodies.

The term "humanized antibody", also known as CDR-grafted antibody, refers to an antibody produced by grafting the CDR sequences of non-human species into the framework of human antibody variable regions. Humanized antibodies can overcome the shortcomings of strong immune responses induced by chimeric antibodies due to the large amount of non-human protein components they carry. In order to avoid the decrease in activity caused by the decrease in immunogenicity, the human antibody variable regions can be subjected to minimal reverse mutation to maintain the activity.

The term "chimeric antibody" is an antibody formed by fusing the antibody variable region of the first species with the antibody constant region of the second species, which can alleviate the immune response induced by an antibody of the first species. As an example, establishing a chimeric antibody requires first establishing a hybridoma that secrets a murine specific monoclonal antibody, then cloning the variable region gene from a murine hybridoma cell, and then cloning the constant region gene of a human antibody as necessary, and linking the murine variable region gene to the human constant region gene to form a chimeric gene, which is inserted into a human expression vector, and finally expressing the chimeric antibody molecule in a eukaryotic industrial system or a prokaryotic industrial system. The constant region of the human antibody can be selected from the heavy chain constant regions of human IgG1, IgG2, IgG3 and IgG4 and variants thereof, preferably comprising a human IgG1, IgG2 or IgG4 heavy chain constant region, or using an IgG1 heavy chain constant region with enhanced ADCC (antibody-dependent cell-mediated cytotoxicity) toxicity after amino acid mutation.

The term "antigen-binding fragment" refers to an antigen-binding fragment of an antibody and an antibody analog, which usually comprises at least part of the antigen-binding region or variable region (for example one or more CDR(s)) of the parental antibody. The antibody fragment retains at least some of the binding specificity of the parental antibody. Typically, when the activity is represented on a mole basis, the antibody fragment retains at least 10% binding activity of the parental antibody. Preferably, the antibody fragment retains at least 20%, 50%, 70%, 80%, 90%, 95% or 100% or more of the binding affinity of the parental antibody to the target. Examples of antigen-binding fragments include, but are not limited to, Fab, Fab', F(ab')2, Fv fragment, linear antibody, single-chain antibody, nanobody, domain antibody and multispecific antibody. Engineered antibody variants are reviewed in Holliger and Hudson, 2005, Nat. Biotechnol. 23: 1126-1136.

The "Fab fragment" consists of one light chain and the CH1 and the variable region of one heavy chain. The heavy chain of a Fab molecule cannot form disulfide bonds with another heavy chain molecule.

The "Fc" region contains two heavy chain fragments comprising the CH1 and CH2 domains of the antibody. The two heavy chain fragments are held together by two or more disulfide bonds and through the hydrophobic interaction of the CH3 domain.

The "Fab' fragment" contains one light chain and a part of one heavy chain comprising the VH domain, the CH1 domain and the region between the CH1 and CH2 domains, so that interchain disulfide bonds can be formed between the two heavy chains of two Fab' fragments, thereby forming a F(ab')2 molecule.

The "F(ab')2 fragment" contains two light chains and two heavy chains comprising a part of the constant region between the CH1 and CH2 domains, thereby forming interchain disulfide bonds between the two heavy chains. Therefore, the F(ab')2 fragment consists of two Fab' fragments held together by disulfide bonds between the two heavy chains.

The "Fv region" comprises variable regions from both the heavy chain and the light chain, but lacks constant regions.

The term "multispecific antibody" is used in its broadest sense, encompassing antibodies with multi-epitope specificity. These multispecific antibodies include, but are not limited to: antibodies comprising heavy chain variable region VH and light chain variable region VL, wherein the VH-VL unit has multi-epitope specificity; antibodies with two or more VL and VH regions, each VH-VL unit binds to a different target or a different epitope of the same target; antibodies with two or more single variable regions, each single variable region binds to a different target or a different epitope of the same target; full-length antibodies, antibody fragments, diabodies, bispecific diabodies and triabodies, covalently or non-covalently linked antibody fragments, and the like.

The term "single-chain antibody" is a single-chain recombinant protein formed by linking the heavy chain variable region VH and light chain variable region VL of an antibody through a linker peptide. It is the minimum antibody fragment with intact antigen binding site.

The term "domain antibody fragment" is an immunoglobulin fragment that contains only the heavy chain variable region or the light chain variable region and has immunological functions. In certain cases, two or more VH regions are covalently linked by peptide linkers to form a bivalent domain antibody fragment. The two VH regions of the bivalent domain antibody fragment can target the same antigen or different antigens.

The term "bind to TROP-2" refers to the ability to interact with TROP-2 or an epitope thereof.

The term "antigen-binding site" refers to a three-dimensional site recognized by the antibody or antigen-binding fragment of the present disclosure.

The term "epitope" refers to a site on an antigen that specifically binds to an immunoglobulin or antibody. Epitopes can be formed by adjacent amino acids or by nonadjacent amino acids by tertiary folding of the protein. Epitopes formed by adjacent amino acids are usually maintained after exposure to a denaturing solvent, while epitopes formed by tertiary folding are usually lost after treatment with a denaturing solvent. Epitopes usually comprise at least 3-15 amino acids in a unique spatial conformation. Methods for determining what epitope is bound to a given antibody are well known in the art, including immunoblotting, immunoprecipitation detection analysis and the like. Methods for determining the spatial conformation of an epitope include the techniques in the art and the techniques described herein, for example X-ray crystallography analysis, two-dimensional nuclear magnetic resonance and the like.

The terms "specific binding" and "selective binding" refer to the binding of an antibody to an epitope on a predetermined antigen. Typically, when recombinant human TROP-2 is used as the analyte and an antibody is used as the ligand, when measured by surface plasmon resonance (SPR) technology in an instrument, the antibody binds to the predetermined antigen (or epitope) at an equilibrium dissociation constant ($K_D$) of about less than $10^{-7}$ M or even less, and its binding affinity to the predetermined antigen (or epitope) is at least two times higher than that for non-specific antigens other than the predetermined antigen or closely related antigens, such as BSA and the like. The term "antigen-recognizing antibody" can be used interchangeably with the term "specific binding antibody" herein.

The term "cross-reaction" refers to the ability of the antibody of the present disclosure to bind to TROP-2 from different species. For example, an antibody of the present disclosure that binds to human TROP-2 can also bind to TROP-2 of another species. Cross-reactivity is measured by detecting the specific reactivity with purified antigen or the binding to or the functional interaction with cells that physiologically express TROP-2 in binding assays (for example SPR and ELISA). Methods for determining cross-reactivity include standard binding assays as described herein, for example surface plasmon resonance (SPR) analysis or flow cytometry.

The terms "inhibition" or "blocking" can be used interchangeably and encompass both partial and complete inhibition/blocking. Preferably, compared to the normal level or type of activity when there is no inhibition or blocking, inhibition or blocking of a ligand reduces or alters the level or type of activity. Inhibition and blocking are also intended to include any measurable reduction in binding affinity to the ligand when in contact with anti-TROP-2 antibody, compared to the ligand not in contact with anti-TROP-2 antibody.

The term "inhibition of growth" (for example involving cells) is intended to include any measurable reduction in cell growth.

The terms "induced immune response" and "enhanced immune response" can be used interchangeably and refer to the immune response stimulated (i.e. passive or adaptive) by a specific antigen. The term "induce" for inducing CDC or ADCC refers to stimulating a specific direct cell killing mechanism.

The term "ADCC", i.e. antibody-dependent cell-mediated cytotoxicity, means that a cell expressing Fc receptors directly kills a target cell targeted by an antibody by recognizing the Fc fragment of the antibody. The ADCC effector function of an antibody can be enhanced, reduced or eliminated by modifying the Fc fragment of IgG. The modification refers to mutation in the heavy chain constant region of the antibody.

The methods for producing and purifying antibodies and antigen-binding fragments are well-known and can be found in the prior art, such as Antibodies: A Laboratory Manual, Cold Spring Harbor, chapters 5-8 and 15. As an example, human TROP-2 or fragment thereof can be used to immunize mice, and the obtained antibodies can be renatured, purified and subjected to amino acid sequencing by conventional methods. Similarly, antigen-binding fragments can also be prepared by conventional methods. One or more human FR regions are added to non-human CDR regions by genetic engineering to obtain the antibody or antigen-binding fragment of the present invention. The human FR germline sequences can be obtained from the ImMunoGeneTics (IMGT) or from The Immunoglobulin FactsBook, 2001ISBN012441351.

The engineered antibodies or antigen-binding fragments of the present disclosure can be prepared and purified by conventional methods. The cDNA sequences of the corresponding antibodies can be cloned and recombined into GS expression vectors. The recombinant immunoglobulin expression vectors can stably transfect CHO cells. As a more recommended prior art, mammalian expression systems can lead to glycosylation of antibodies, especially at the highly conserved N-terminus of the Fc region. Stable clones are obtained by expressing antibodies that specifically bind to human antigens. Positive clones are expanded in serum-free medium in bioreactors to produce the antibodies. The culture fluid into which the antibodies are secreted can be purified and collected by conventional techniques. The antibodies can be filtered and concentrated by conventional methods. Soluble mixtures and multimers can also be removed by conventional methods, for example molecular sieves and ion exchange. The resulting product needs to be frozen immediately, such as at -70°C, or lyophilized.

The antibody of the present disclosure refers to monoclonal antibody. The monoclonal antibody (mAb) as described the present disclosure refers to an antibody obtained from a monoclonal cell strain, which is not limited to eukaryotic, prokaryotic or phage clonal cell strains. Monoclonal antibodies or antigen-binding fragments can be obtained by recombination using, for example, hybridoma technology, recombination technology, phage display technology, synthesis technology (such as CDR-grafting) or other existing technologies.

"Administration", "dosing" and "treatment", when applied to an animal, a human, an experimental subject, a cell, a tissue, an organ or a biological fluid, refer to contacting an exogenous medicament, a therapeutic agent, a diagnostic agent or a composition with the animal, human, subject, cell, tissue, organ or biological fluid. "Administration", "dosing" and "treatment" can refer to, for example, treatment, pharmacokinetics, diagnostic, research and experimental methods. Treatment of a cell includes contacting a reagent with the cell, and contacting the reagent with a fluid, wherein the fluid is in contact with the cell. "Administration", "dosing" and "treatment" also refer to treating, for example a cell, by a reagent, diagnostic, a binding composition or by another cell *in vitro* and *ex vivo*. "Administration", "dosing" and "treatment", when applied to a human, a veterinary or research subject, refer to therapeutic treatment, preventive or prophylactic measures, research and diagnostic applications.

"Treatment" refers to providing an internal or external therapeutic agent (for example agent comprising any one of the antibodies or a fragment thereof of the present disclosure) to a subject having one or more disease symptoms on which the therapeutic agent is known to have therapeutic effect. Typically, the therapeutic agent is given at an amount effective to alleviate one or more disease symptoms in the subject or subject population being treated, either to induce the regression of such symptoms, or to suppress the progression of such symptoms to any clinically measurable extent. The amount of therapeutic agent effective to alleviate any specific disease symptom (also referred to as a "therapeutically effective amount") can vary depending on a variety of factors, for example the subject's disease state, age and body weight, and the ability of the drug to produce the desired therapeutic effect in the subject. Whether the disease symptom has been alleviated can be evaluated by any clinical testing method commonly used by doctors or other health care professionals for evaluating the severity or progression of the symptom. Although the embodiments of the present disclosure (for example a treatment method or a product) may not be effective in alleviating the target disease symptom in every subject, but as determined according to any statistical test methods known in the art such as Student t test, chi-square test, Mann and Whitney's U test, Kruskal-Wallis test (H test), Jonckheere-Terpstra test and Wilcoxon test, they should significantly reduce the target disease symptom in a statistically effective number of subjects.

The "effective amount" includes an amount sufficient to ameliorate or prevent a medical condition or a symptom thereof. The effective amount also refers to an amount sufficient to allow or facilitate diagnosis. The effective amount for a particular subject or veterinary subject can vary depending on the following factors, such as, the condition to be treated, the general health condition of the subject, the method, route and dosage of dosing, and the severity of side effects. The effective amount can be the maximum dose or dosing regimen that avoids significant side effects or toxic effects.

"Exogenous" refers to substances produced outside organisms, cells or human bodies depending on backgrounds.

"Endogenous" refers to substances produced inside cells, organisms or human bodies depending on backgrounds.

"Homology" or "identity" refers to the sequence similarity between two polynucleotide sequences or between two polypeptides. When the positions in the two sequences aligned are occupied by the same base or amino acid residue, for example if each position of two DNA molecules is occupied by adenine, then the molecules are homologous at that position. The homology percentage between two sequences is a function of the number of matched or homologous positions shared among the two sequences divided by the number of positions aligned × 100%. For example, in the optimal sequence alignment, if 6 out of 10 positions in the two sequences are matched or homologous, then the two sequences are 60% homologous. Generally, the alignment is made when two sequences are aligned to obtain the maximum homology percentage.

The expressions "cell", "cell line" and "cell culture" used herein can be used interchangeably, and all such names include progenies thereof. Therefore, the expressions "transformant" and "transformed cell" include primary cells and cultures derived therefrom, regardless of the number of passages. It should also be understood that due to intentionalor unintentional mutations, it is impossible that all progenies are exactly the same in terms of DNA content. Mutant progenies with the same function or biological activity as screened in the primary cells are included. It can be clearly seen from the context when different names are referred to.

"Optional" or "optionally" means that the event or circumstance described subsequently can occur but need not occur, and the description includes the cases in which the event or circumstance occurs or does not occur. For example, "optionally comprising 1 to 3 antibody heavy chain variable regions" means that the antibody heavy chain variable regions of particular sequences can but need not be present.

The "pharmaceutical composition" means it contains one or more antibodies or antigen-binding fragments described herein as well as other components such as physiological/pharmaceutically acceptable carriers and excipients. The objective of the pharmaceutical composition is to facilitate drug administration to an organism, and to benefit the absorption of the active ingredient so as to exert the biological activity.

In the context, if not clearly indicated, the singular form "a/an", "one" or "the" also includes its corresponding plural form.

The present disclosure will be further described with reference to the following examples, but the examples should not be considered as limiting the scope of the present disclosure. The experimental methods without specified conditions in the examples of the present disclosure usually follow conventional conditions, such as those in Antibodies: A Laboratory Manual and Molecular Cloning: A Laboratory Manual, Cold Spring Harbor; or according to the conditions recommended by the raw material or product manufacturer. The reagents without specified sources are conventional reagents purchased from the market.

## Example 1: Preparation of antigens

The protein encoding the His-tagged human TROP-2 (TROP-2-His) was synthesized by SinoBiologics (10428-H08H).

The sequence of TROP-2-His:

MARGPGLAPPPLRLPLLLLVLAAVTGHTAAQDNCTCPTNKMTVCSPDGPGGRC
QCRALGSGMAVDCSTLTSKCLLLKARMSAPKNARTLVRPSEHALVDNDGLYDP
DCDPEGRFKARQCNQTSVCWCVNSVGVRRTDKGDLSLRCDELVRTHHILIDLR
HRPTAGAFNHSDLDAELRRLFRERYRLHPKFVAAVHYEQPTIQIELRQNTSQKA
AGDVDIGDAAYYFERDIKGESLFQGRGGLDLRVRGEPLQVERTLIYYLDEIPPKF
SMKRLTAGLIAVIVVVVVALVAGMAVLVITNRRKSGKYKKVEIKELGELRKEPSL

HHHHHHHH

SEQ ID NO: 1.

## Example 2: Obtaining murine hybridomas and antibody sequences

A total of 5 Balb/c and 5 A/J female 10-week-old mice were immunized with the human antigen TROP-2-His. Sigma Complete Freund's Adjuvant (CFA) and Sigma Incomplete Freund's Adjuvant (IFA) were used. The immunogen and the immune adjuvant were thoroughly mixed at a ratio of 1:1 and emulsified to make a stable "water-in-oil" liquid. The injection dose was 25 $\mu$g/200 $\mu$L/mouse.

Table 1. Immunization scheme

| Day 1 | First immunization, complete Freund's adjuvant. |
|---|---|
| Day 21 | Second immunization, incomplete Freund's adjuvant. |
| Day 35 | Third immunization, incomplete Freund's adjuvant. |
| Day 42 | Blood sampling and serum titer test (blood after 3 immunizations) |
| Day 49 | Fourth immunization, incomplete Freund's adjuvant. |
| Day 56 | Blood sampling and serum titer test (blood after 4 immunizations) |

As for the serum of immunized mice, the serum titer and the ability to bind to cell surface antigens were evaluated by using indirect ELISA as described in Example 3. The initiation of cell fusion was determined according to the detection results of titer (greater than 100,000-fold dilution). The immunized mice with high serum titer, affinity and FACS binding were selected for one final immunization and then sacrificed. The spleen cells were collected, fused with SP2/0 myeloma cells and plated to obtain hybridomas. The target hybridomas were screened by indirect ELISA and established as monoclonal cell strains by limiting dilution method. The obtained positive antibody strains were further screened by indirect ELISA so as to select the hybridomas that bound to the recombinant protein. Hybridoma cells at logarithmic growth phase were collected. RNA was extracted with Trizol (Invitrogen, 15596-018) and subjected to reverse transcription (PrimeScript™ Reverse Transcriptase, Takara #2680A). The cDNA obtained by reverse transcription was amplified by PCR using a mouse Ig-primer set (Novagen, TB326 Rev.B 0503) and subjected to sequencing to finally obtain the sequence of the murine antibody.

The sequences of the heavy chain and light chain variable regions of murine monoclonal antibody M1 are as follows:

M1 HCVR

QVQLQQPGAELVRPGASVKLSCRASGYTFT<u>NYWMN</u>WVKQRPEQGLEWIG<u>RID
PNDSETHYNQKFKD</u>RAILTVDKASNTAYMQLSGLTSEDSAVHYCAR<u>SGFGSTY
WFFDV</u>WGAGTTVTVSS

SEQ ID NO: 2

M1 LCVR

DIVMTQSHKFMSTSVGDRVSITC<u>KASQDVSTAVA</u>WYQQKPGQSPKLLIY<u>SASYR</u>

<u>YT</u>GVPDRFAGSGYGTDFTFTISSVQTEDLTVYHC<u>QQHYSTPLT</u>FGPGTRLELK

SEQ ID NO: 3.

Table 2. The CDR sequences of the heavy chain and light chain variable regions of murine monoclonal antibody M1

| Name | Sequence | No. |
|---|---|---|
| HCDR1 | NYWMN | SEQ ID NO: 4 |
| HCDR2 | RIDPNDSETHYNQKFKD | SEQ ID NO: 5 |
| HCDR3 | SGFGSTYWFFDV | SEQ ID NO: 6 |
| LCDR1 | KASQDVSTAVA | SEQ ID NO: 7 |
| LCDR2 | SASYRYT | SEQ ID NO: 8 |
| LCDR3 | QQHYSTPLT | SEQ ID NO: 9 |

**Examples 3: Detection method for *in vitro* binding activity of antibodies**

*In vitro* indirect ELISA binding experiment:
TROP-2 His protein (Sino Biological Inc., cat# 10428-H08H) was diluted with pH 7.4 PBS to a concentration of 1 $\mu$g/ml, added to a 96-well high-affinity ELISA plate at 100 $\mu$l/well and incubated in a refrigerator at 4°C overnight (16-20 hours). After washing the plate 4 times with PBST (pH 7.4 PBS comprising 0.05% Tween-20), 3% bovine serum albumin (BSA) blocking solution diluted with PBST was added at 150 $\mu$l/well and incubated at room temperature for 1 hour for blocking. After completion of the blocking, the blocking solution was discarded, and the plate was washed 4 times with PBST buffer.

The antibody to be tested was diluted with PBST comprising 3% BSA to obtain a series of 5-fold dilutions (with 9 doses in total, starting from 10 $\mu$M), and was added to the ELISA plate at 100 $\mu$l/well, and incubated at room temperature for 1 hour. After completion of the incubation, the plate was washed 4 times with PBST. HRP-labeled goatanti-human secondary antibody (Abcam, cat#ab97225) diluted with PBST comprising 3% BSA was added at 100 $\mu$l/well and incubated at room temperature for 1 hour. The plate was washed 4 times with PBST, then TMB chromogenic substrate (Cell Signaling Technology, cat#7004S) was added at 100 $\mu$l/well and incubated at room temperature in the dark for 1 minute. The stop solution (Cell Signaling Technology, cat#7002S) was added at 100 $\mu$l/well to terminate the reaction. The absorbance at 450 nm was read with a microplate reader (BioTek, model Synergy H1) and the data were analyzed. The results were analyzed by plotting the concentration-signal curve, as shown in Table 3 below:

Table 3. Affinity of murine antibodies to human TROP-2 antigen ($EC_{50}$ value)

| Murine antibody | $EC_{50}$ representing the binding to human TROP-2 His antigen (nM) |
|---|---|
| M1 | 0.56 |

**Example 4: Humanization experiment of murine antibodies**

Humanization of the murine anti-human TROP-2 monoclonal antibodies was performed by using methods as disclosed in many literatures in the art. Briefly, parental (murine antibody) constant domains were replaced with human constant domains. Human germline antibody sequences were selected according to the homology of the murine antibody and human antibody. The murine antibody M1 was humanized in the present disclosure.

On the basis of the typical structure of the VH/VL CDR of the obtained murine antibody, the sequences of heavy and light chain variable regions were aligned with the human antibody germline database to obtain human germline templates with high homology.

The CDR regions of the murine antibody M1 were grafted onto the selected corresponding humanizing templates. Then, based on the three-dimensional structure of the murine antibody, the embedded residues, the residues directly interacting with the CDR regions and the residues with significant influence on the conformation of VL and VH were subjected to back mutation. After expression test and comparison of the number of back mutations, the antibody designed by combining humanized heavy chain variable region HCVR and light chain variable region LCVR sequences was selected, and the sequences are as follows:

HU6 HCVR

EVQLLESGGGLVQPGGSLRLSCAASGFTVSNYWMNWVRQAPGKGLEWMGRID
PNDSETHYNQKFKDRVTISVDKSKNQFSLKLSSVTAADTAVYYCARSGFGSTYW
FFDVWGQGTTVTVSS

SEQ ID NO: 10

HU6 LCVR

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIK

SEQ ID NO: 11.

The designed sequences of heavy chain and light chain variable regions were linked to the sequences of IgG1 heavy chain and light chain constant regions respectively. The sequence of the linked human IgG1 heavy chain constant region is as follows:

IgG1 C1

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPELLGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK

SEQ ID NO: 12;

the designed sequences of heavy chain and light chain variable regions were linked to the sequences of IgG1 heavy chain and light chain constant regions respectively. The sequence of the linked human kappa chain constant region is as follows:
Ig kappa C

RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

SEQ ID NO: 13;

exemplary heavy chain and light chain sequences obtained after linking are as follows:
HU6DL HC

EVQLLESGGGLVQPGGSLRLSCAASGFTVSNYWMNWVRQAPGKGLEWMGRID PNDSETHYNQKFKDRVTISVDKSKNQFSLKLSSVTAADTAVYYCARSGFGSTYW FFDVWGQGTTVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTKV DKKVEPKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWLNGKE YKCKVSNKALPAPIEKTISKAKGQPREPQVYTLPPSRDELTKNQVSLTCLVKGFY PSDIAVEWESNGQPENNYKTTPPVLDSDGSFFLYSKLTVDKSRWQQGNVFSCSV MHEALHNHYTQKSLSLSPGK

SEQ ID NO: 14

HU6DL LC

DVVMTQSPLSLPVTLGQPASISCKASQDVSTAVAWYQQKPGKAPKLFIYSASYR YTGVPSRFSGSGSGTDFTLTISSLQPEDFATYYCQQHYSTPLTFGQGTRLEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC

SEQ ID NO: 15.

**Example 5: HU6DL variant design experiment**

The humanized antibody HU6DL has an $N^{54}D^{55}S^{56}$ motif in HCDR2 and is susceptible to glycosylation. N54 was subjected to site-directed mutation by computeraided technology to reduce the potential risk of glycosylation without affecting its binding to antigen and its own thermostability. The mutants of antibody HU6DL, namely HU6DL.R54, HU6DL.Y54, HU6DL.Q54, HU6DL.L54, HU6DL.T54, HU6DL.I54, HU6DL.F54, HU6DL.E54 and HU6DL.A54, were obtained, and the corresponding heavy chain and light chain variable regions are as follows:

HU6DL.R54 HCVR

EVQLLESGGGLVQPGGSLRLSCAASGFTVSNYWMNWVRQAPGKGLEWMGRID PRDSETHYNQKFKDRVTISVDKSKNQFSLKLSSVTAADTAVYYCARSGFGSTYW FFDVWGQGTTVTVSS

SEQ ID NO: 16

HU6DL.R54 LCVR

DVVMTQSPLSLPVTLGQPASISCKASQDVSTAVAWYQQKPGKAPKLFIYSASYR YTGVPSRFSGSGSGTDFTLTISSLQPEDFATYYCQQHYSTPLTFGQGTRLEIK

SEQ ID NO: 11

HU6DL.Y54 HCVR

EVQLLESGGGLVQPGGSLRLSCAASGFTVSNYWMNWVRQAPGKGLEWMGRID

PYDSETHYNQKFKDRVTISVDKSKNQFSLKLSSVTAADTAVYYCARSGFGSTYW
FFDVWGQGTTVTVSS

SEQ ID NO: 17

HU6DL.Y54 LCVR

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIK

SEQ ID NO: 11

HU6DL.Q54 HCVR

EVQLLESGGGLVQPGGSLRLSCAASGFTVSNYWMNWVRQAPGKGLEWMGRID
PQDSETHYNQKFKDRVTISVDKSKNQFSLKLSSVTAADTAVYYCARSGFGSTYW
FFDVWGQGTTVTVSS

SEQ ID NO: 18

HU6DL.Q54 LCVR

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIK

SEQ ID NO: 11

HU6DL.L54 HCVR

EVQLLESGGGLVQPGGSLRLSCAASGFTVSNYWMNWVRQAPGKGLEWMGRID
PLDSETHYNQKFKDRVTISVDKSKNQFSLKLSSVTAADTAVYYCARSGFGSTYW
FFDVWGQGTTVTVSS

SEQ ID NO: 19

HU6DL.L54 LCVR

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIK

SEQ ID NO: 11

HU6DL.T54 HCVR

EVQLLESGGGLVQPGGSLRLSCAASGFTVSNYWMNWVRQAPGKGLEWMGRID
PTDSETHYNQKFKDRVTISVDKSKNQFSLKLSSVTAADTAVYYCARSGFGSTYW
FFDVWGQGTTVTVSS

SEQ ID NO: 20

HU6DL.T54 LCVR

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIK

SEQ ID NO: 11

HU6DL.I54 HCVR

EVQLLESGGGLVQPGGSLRLSCAASGFTVSNYWMNWVRQAPGKGLEWMGRID
PIDSETHYNQKFKDRVTISVDKSKNQFSLKLSSVTAADTAVYYCARSGFGSTYW
FFDVWGQGTTVTVSS

SEQ ID NO: 31

HU6DL.I54 LCVR

DVVMTQSPLSLPVTLGQPASISCKASQDVSTAVAWYQQKPGKAPKLFIYSASYR

YTGVPSRFSGSGSGTDFTLTISSLQPEDFATYYCQQHYSTPLTFGQGTRLEIK

SEQ ID NO: 11

HU6DL.F54 HCVR

EVQLLESGGGLVQPGGSLRLSCAASGFTVSNYWMNWVRQAPGKGLEWMGRID
PFDSETHYNQKFKDRVTISVDKSKNQFSLKLSSVTAADTAVYYCARSGFGSTYW
FFDVWGQGTTVTVSS

SEQ ID NO: 32

HU6DL.F54 LCVR

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIK

SEQ ID NO: 11

HU6DL.E54 HCVR

EVQLLESGGGLVQPGGSLRLSCAASGFTVSNYWMNWVRQAPGKGLEWMGRID
PEDSETHYNQKFKDRVTISVDKSKNQFSLKLSSVTAADTAVYYCARSGFGSTYW
FFDVWGQGTTVTVSS

SEQ ID NO: 33

HU6DL.E54 LCVR

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIK

SEQ ID NO: 11

HU6DL.A54 HCVR

EVQLLESGGGLVQPGGSLRLSCAASGFTVSNYWMNWVRQAPGKGLEWMGRID
PADSETHYNQKFKDRVTISVDKSKNQFSLKLSSVTAADTAVYYCARSGFGSTYW
FFDVWGQGTTVTVSS

SEQ ID NO: 34

HU6DL.A54 LCVR

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIK

SEQ ID NO: 11.

The designed sequences of heavy chain and light chain variable regions were linked to the sequences of IgG1 heavy chain constant region and light chain constant region respectively. Exemplary heavy chain and light chain sequences obtained are as follows (wherein, HU6DL.R54, HU6DL.Y54, HU6DL.Q54, HU6DL.L54, HU6DL.T54, HU6DL.I54, HU6DL.F54, HU6DL.E54 and HU6DL.A54 heavy chains result from linking the sequences SEQ ID NO: 16, SEQ ID NO: 17, SEQ ID NO: 18, SEQ ID NO: 19, SEQ ID NO: 20, SEQ ID NO: 31, SEQ ID NO: 32, SEQ ID NO: 33, and SEQ ID NO: 34 to the sequence SEQ ID NO: 12 respectively):

HU6DL.R54 HC

EVQLLESGGGLVQPGGSLRLSCAASGFTVSNYWMNWVRQAPGKGLEWMGRID
PRDSETHYNQKFKDRVTISVDKSKNQFSLKLSSVTAADTAVYYCARSGFGSTYW

FFDVWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK

SEQ ID NO: 21

HU6DL.R54 LC

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 15

HU6DL.Y54 HC

EVQLLESGGGLVQPGGSLRLSCAASGFTVSNYWMNWVRQAPGKGLEWMGRID PYDSETHYNQKFKDRVTISVDKSKNQFSLKLSSVTAADTAVYYCARSGFGSTYW FFDVWGQGTTVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTKV DKKVEPKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWLNGKE YKCKVSNKALPAPIEKTISKAKGQPREPQVYTLPPSRDELTKNQVSLTCLVKGFY PSDIAVEWESNGQPENNYKTTPPVLDSDGSFFLYSKLTVDKSRWQQGNVFSCSV MHEALHNHYTQKSLSLSPGK

SEQ ID NO: 22

HU6DL.Y54 LC

DVVMTQSPLSLPVTLGQPASISCKASQDVSTAVAWYQQKPGKAPKLFIYSASYR YTGVPSRFSGSGSGTDFTLTISSLQPEDFATYYCQQHYSTPLTFGQGTRLEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC

SEQ ID NO: 15

HU6DL.Q54 HC

EVQLLESGGGLVQPGGSLRLSCAASGFTVSNYWMNWVRQAPGKGLEWMGRID PQDSETHYNQKFKDRVTISVDKSKNQFSLKLSSVTAADTAVYYCARSGFGSTYW FFDVWGQGTTVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTKV DKKVEPKSCDKTHTCPPCPAPELLGGPSVFLFPPKPKDTLMISRTPEVTCVVVDV SHEDPEVKFNWYVDGVEVHNAKTKPREEQYNSTYRVVSVLTVLHQDWLNGKE YKCKVSNKALPAPIEKTISKAKGQPREPQVYTLPPSRDELTKNQVSLTCLVKGFY PSDIAVEWESNGQPENNYKTTPPVLDSDGSFFLYSKLTVDKSRWQQGNVFSCSV MHEALHNHYTQKSLSLSPGK

SEQ ID NO: 23

HU6DL.Q54 LC

DVVMTQSPLSLPVTLGQPASISCKASQDVSTAVAWYQQKPGKAPKLFIYSASYR YTGVPSRFSGSGSGTDFTLTISSLQPEDFATYYCQQHYSTPLTFGQGTRLEIKRTV AAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC

SEQ ID NO: 15

HU6DL.L54 HC

EVQLLESGGGLVQPGGSLRLSCAASGFTVSNYWMNWVRQAPGKGLEWMGRID
PLDSETHYNQKFKDRVTISVDKSKNQFSLKLSSVTAADTAVYYCARSGFGSTYW
FFDVWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK

SEQ ID NO: 24

HU6DL.L54 LC

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 15

HU6DL.T54 HC

EVQLLESGGGLVQPGGSLRLSCAASGFTVSNYWMNWVRQAPGKGLEWMGRID
PTDSETHYNQKFKDRVTISVDKSKNQFSLKLSSVTAADTAVYYCARSGFGSTYW
FFDVWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK

SEQ ID NO: 25

HU6DL.T54 LC

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 15

HU6DL.I54 HC

EVQLLESGGGLVQPGGSLRLSCAASGFTVSNYWMNWVRQAPGKGLEWMGRID
PIDSETHYNQKFKDRVTISVDKSKNQFSLKLSSVTAADTAVYYCARSGFGSTYW
FFDVWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK

SEQ ID NO: 35

HU6DL.I54 LC

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 15

HU6DL.F54 HC

EVQLLESGGGLVQPGGSLRLSCAASGFTVSNYWMNWVRQAPGKGLEWMGRID
PFDSETHYNQKFKDRVTISVDKSKNQFSLKLSSVTAADTAVYYCARSGFGSTYW
FFDVWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK

SEQ ID NO: 36

HU6DL.F54 LC

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 15

HU6DL.E54 HC

EVQLLESGGGLVQPGGSLRLSCAASGFTVSNYWMNWVRQAPGKGLEWMGRID
PEDSETHYNQKFKDRVTISVDKSKNQFSLKLSSVTAADTAVYYCARSGFGSTYW
FFDVWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE

YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK

SEQ ID NO: 37

HU6DL.E54 LC

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 15

HU6DL.A54 HC

EVQLLESGGGLVQPGGSLRLSCAASGFTVSNYWMNWVRQAPGKGLEWMGRID
PADSETHYNQKFKDRVTISVDKSKNQFSLKLSSVTAADTAVYYCARSGFGSTYW
FFDVWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK

SEQ ID NO: 38

HU6DL.A54 LC

DVVMTQSPLSLPVTLGQPASISCKASQDVSTAVAWYQQKPGKAPKLFIYSASYR
YTGVPSRFSGSGSGTDFTLTISSLQPEDFATYYCQQHYSTPLTFGQGTRLEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

SEQ ID NO: 15.

Table 4. The sequences of heavy chain variable region HCDR2 of HU6DL mutants

| Name | Sequence | No. |
|---|---|---|
| HU6DL.R54 | RIDPRDSETHYNQKFKD | SEQ ID NO: 26 |
| HU6DL.Y54 | RIDPYDSETHYNQKFKD | SEQ ID NO: 27 |
| HU6DL.Q54 | RIDPQDSETHYNQKFKD | SEQ ID NO: 28 |
| HU6DL.L54 | RIDPLDSETHYNQKFKD | SEQ ID NO: 29 |
| HU6DL.T54 | RIDPTDSETHYNQKFKD | SEQ ID NO: 30 |
| HU6DL.I54 | RIDPIDSETHYNQKFKD | SEQ ID NO: 39 |
| HU6DL.F54 | RIDPFDSETHYNQKFKD | SEQ ID NO: 40 |
| HU6DL.E54 | RIDPEDSETHYNQKFKD | SEQ ID NO: 41 |
| HU6DL.A54 | RIDPADSETHYNQKFKD | SEQ ID NO: 42 |

Table 5. Sequence Nos. of HU6DL mutants

| Name | Heavy chain/ light chain No. | Heavy chain/light chain variable region No. | Heavy chain/light chain CDR1 No. | Heavy chain/light chain CDR2 No. | Heavy chain/light chain CDR3 No. |
|---|---|---|---|---|---|
| HU6DL.R54 | SEQ ID NO: 21 (heavy chain) | SEQ ID NO: 16 (heavy chain variable region) | SEQ ID NO: 4 | SEQ ID NO: 26 | SEQ ID NO: 6 |
|  | SEQ ID NO: 15 (light chain) | SEQ ID NO: 11 (light chain variable region) | SEQ ID NO: 7 | SEQ ID NO: 8 | SEQ ID NO: 9 |
| HU6DL.Y54 | SEQ ID NO: 22 (heavy chain) | SEQ ID NO: 17 (heavy chain variable region) | SEQ ID NO: 4 | SEQ ID NO: 27 | SEQ ID NO: 6 |
|  | SEQ ID NO: 15 (light chain) | SEQ ID NO: 11 (light chain variable region) | SEQ ID NO: 7 | SEQ ID NO: 8 | SEQ ID NO: 9 |
| HU6DL.Q54 | SEQ ID NO: 23 (heavy chain) | SEQ ID NO: 18 (heavy chain variable region) | SEQ ID NO: 4 | SEQ ID NO: 28 | SEQ ID NO: 6 |
|  | SEQ ID NO: 15 (light chain) | SEQ ID NO: 11 (light chain variable region) | SEQ ID NO: 7 | SEQ ID NO: 8 | SEQ ID NO: 9 |
| HU6DL.L54 | SEQ ID NO: 24 (heavy chain) | SEQ ID NO: 19 (heavy chain variable region) | SEQ ID NO: 4 | SEQ ID NO: 29 | SEQ ID NO: 6 |
|  | SEQ ID NO: 15 (light chain) | SEQ ID NO: 11 (light chain variable region) | SEQ ID NO: 7 | SEQ ID NO: 8 | SEQ ID NO: 9 |
| HU6DL.T54 | SEQ ID NO: 25 (heavy chain) | SEQ ID NO: 20 (heavy chain variable region) | SEQ ID NO: 4 | SEQ ID NO: 30 | SEQ ID NO: 6 |
|  | SEQ ID NO: 15 (light chain) | SEQ ID NO: 11 (light chain variable region) | SEQ ID NO: 7 | SEQ ID NO: 8 | SEQ ID NO: 9 |

(continued)

| Name | Heavy chain/light chain No. | Heavy chain/light chain variable region No. | Heavy chain/light chain CDR1 No. | Heavy chain/light chain CDR2 No. | Heavy chain/light chain CDR3 No. |
|---|---|---|---|---|---|
| HU6DL.I54 | SEQ ID NO: 35 (heavy chain) | SEQ ID NO: 31 (heavy chain variable region) | SEQ ID NO: 4 | SEQ ID NO: 39 | SEQ ID NO: 6 |
| | SEQ ID NO: 15 (light chain) | SEQ ID NO: 11 (light chain variable region) | SEQ ID NO: 7 | SEQ ID NO: 8 | SEQ ID NO: 9 |
| HU6DL.F54 | SEQ ID NO: 36 (heavy chain) | SEQ ID NO: 32 (heavy chain variable region) | SEQ ID NO: 4 | SEQ ID NO: 40 | SEQ ID NO: 6 |
| | SEQ ID NO: 15 (light chain) | SEQ ID NO: 11 (light chain variable region) | SEQ ID NO: 7 | SEQ ID NO: 8 | SEQ ID NO: 9 |
| HU6DL.E54 | SEQ ID NO: 37 (heavy chain) | SEQ ID NO: 33 (heavy chain variable region) | SEQ ID NO: 4 | SEQ ID NO: 41 | SEQ ID NO: 6 |
| | SEQ ID NO: 15 (light chain) | SEQ ID NO: 11 (light chain variable region) | SEQ ID NO: 7 | SEQ ID NO: 8 | SEQ ID NO: 9 |
| HU6DL.A54 | SEQ ID NO: 38 (heavy chain) | SEQ ID NO: 34 (heavy chain variable region) | SEQ ID NO: 4 | SEQ ID NO: 42 | SEQ ID NO: 6 |
| | SEQ ID NO: 15 (light chain) | SEQ ID NO: 11 (light chain variable region) | SEQ ID NO: 7 | SEQ ID NO: 8 | SEQ ID NO: 9 |

The cDNA fragments were synthesized according to the amino acid sequences of the above humanized antibody light chain and heavy chains. Protein expression services were provided by Beijing Sinobiological Co., Ltd. HEK293 cells were transiently transfected with HU6DL protein mutants for expression The antibodies were detected for their purity by using molecular exclusion chromatography, and their concentration and purity were as shown in Table 6 below.

Table 6. Concentration and purity of HU6DL mutants

| Name | Concentration (mg/mL) | Purity (%) |
|---|---|---|
| HU6DL.R54 | 1.99 | 95.8 |
| HU6DL.Y54 | 1.34 | 96.4 |
| HU6DL.Q54 | 1.73 | 93.1 |
| HU6DL.L54 | 1.36 | 94.9 |
| HU6DL.T54 | 2.73 | 96.1 |
| HU6DL.I54 | 2.00 | 95.5 |
| HU6DL.F54 | 1.68 | 93.5 |
| HU6DL.E54 | 1.56 | 95.6 |
| HU6DL.A54 | 1.00 | 95.1 |

**Example 6. Affinity experiment of HU6DL mutants to TROP-2 antigen**

1. Objective of the experiment:

The difference in affinity level of different anti-TROP-2 mutants to TROP-2 antigen was evaluated by sandwich ELISA, i.e., "antigen-antibody-HRP-labeled secondary antibody".

2. Experimental steps:

The his-tagged TROP-2 protein (SinoBiologics, Cat: 10428-H08H) was diluted to 1 $\mu$g/mL using DPBS pH 7.4, and added to a high-affinity 96-well plate (Corning, Cat: 3590) at 100 $\mu$L/well and incubated overnight at 4°C. On the next day, the TROP-2 antigen solution was discarded. PBS pH 7.4 comprising 0.05% Tween 20 (PBST) solution was added at 200 $\mu$L/well for washing 3 times. 2% BSA (dissolved in PBST) was added at 200 $\mu$L/well for blocking at 37°C for 1 h and the plate was washed 3 times with PBST. Serial dilutions of the candidate antibody (10-fold dilutions giving 8 concentration levels from 10 nM to $1 \times 10^{-6}$ nM) were added at 100 $\mu$L/well, with 0.5% BSA used as a negative control, and incubated at 37°C for 1 h for blocking. The plate was washed 3 times with PBST. Goat anti-human IgG, Fc-HRP (Abcam, cat: ab97225) secondary antibody solution diluted at 1: 10000 was added at 100 $\mu$L/well and incubated at 37°C for 1 h for blocking. The plate was washed 3 times with PBST.

TMB (CST, Cat: 7004P6) substrate was added at 100 $\mu$L/well for reaction at room temperature for 3 min, until the color of the solution in the well with highest antibody concentration changed to dark blue. Stop solution (CST, Cat: 7002P6) was added at 50 $\mu$L/well to terminate the reaction. The absorbance (OD) was read at a wavelength of 450 nm.

3. Data processing:

The $EC_{50}$ (the affinity of each HU6DL mutant to the antigen) was calculated by plotting, with the logarithmic concentration of the candidate antibody as the X-coordinate and the OD450 absorbance as the Y-coordinate, and fitting against log (agonist) vs. response-viable slope (four-parameter) equation in GraphPad PRISM 8.0. The affinity ($EC_{50}$) of HU6DL mutants to human TROP-2 antigen was as shown in Table 7 below:

Table 7. Affinity of HU6DL mutants to human TROP-2 antigen ($EC_{50}$)

| Name | TOP OD$_{450}$ | Affinity EC$_{50}$ (nM) |
| --- | --- | --- |
| HU6DL.R54 | 2.05 | 0.052 |
| HU6DL.Y54 | 1.93 | 0.051 |
| HU6DL.Q54 | 1.91 | 0.086 |
| HU6DL.L54 | 1.96 | 0.048 |
| HU6DL.T54 | 1.90 | 0.052 |
| HU6DL.I54 | 1.96 | 0.063 |
| HU6DL.F54 | 1.93 | 0.067 |
| HU6DL.E54 | 1.96 | 0.064 |
| HU6DL.A54 | 2.07 | 0.062 |

4. Conclusion of the experiment:

The above data show that each HU6DL mutant of the present disclosure has good affinity to human TROP-2 antigen.

**Example 7: Affinity experiment of HU6DL mutants on tumor cells**

1. Objective of the experiment:
The difference in affinity levels of HU6DL mutants to tumor cell lines expressing TROP-2 antigen was evaluated by flow cytometry.
2. Experimental reagents:

Gastric cancer cell NCI-N87 (purchased from Chinese Academy of Sciences Cell Bank, TCHu130);
non-small cell lung cancer cell HCC827 (purchased from Chinese Academy of Sciences Cell Bank, TCHu153);
bladder cancer cell SW780 (purchased from Chinese Academy of Sciences Cell Bank, TCHu219);
bladder cancer cell RT4 (purchased from Chinese Academy of Sciences Cell Bank, TCHu226).

3. Experimental steps:

Tumor cells in a well-growing state were treated with Accutase (Sigma, cat: A6964) digestion solution. Single-cell suspension was prepared in 2% FBS (diluted in DBPS, pH 7.4) solution and the cell concentration was adjusted to $1 \times 10^6$ cells/mL. The suspension was aliquoted into a 96-well V-shaped bottom plate at 100 μL/well and centrifuged at 300 g × 5 min at 4°C. The supernatant was discarded, and serial dilution of the candidate antibody solution (10-fold dilution giving 10 concentration levels from 1000 nM to $1 \times 10^{-6}$ nM) was added at 100 μL/well and incubated at 4°C for 1 h.

The plate was centrifuged at 300 g × 5 min at 4°C and washed twice. Mouse-anti-human IgG Fc, PE-labeled secondary antibody (Biolegend, cat: 409304) solution diluted at a ratio of 5 μL/$10^6$ cells was added at 100 μL/well and incubated at 4°C for 1 h. The plate was centrifuged at 300 g × 5 min at 4°C and washed twice. 70 μL of 2% FBS solution was added to resuspend the cells. The mean fluorescence intensity (MFI) of the PE channel was detected by a ZE5 flow cytometry (Bio-Rad, ZE5).

4. Data processing:

The $EC_{50}$ (the affinity of each candidate antibody to the tumor cells) was calculated by plotting, with the logarithmic concentration of the mutant antibody as the X-coordinate and the MFI as the Y-coordinate and fitting against log (agonist) vs. response-viable slope (four-parameter) equation in GraphPad PRISM 8.0, as shown in Table 8 below:

Table 8. Affinity of HU6DL mutants to NCI-N87, HCC827, SW780 and RT4 tumor cells ($EC_{50}$)

| Name | NCI-N87 | | HCC827 | | SW780 | | RT4 | |
|---|---|---|---|---|---|---|---|---|
| | TOP MFI | $EC_{50}$ (nM) | TOP MFI | $EC_{50}$ (nM) | TOP MFI | $EC_{50}$ (nM) | TOP MFI | $EC_{50}$ (nM) |
| HU6DL.R54 | 2693 | 1.63 | 6088 | 1.36 | 6581 | 1.22 | 3231 | 1.21 |
| HU6DL.T54 | 1703 | 2.25 | 4563 | 1.46 | 4850 | 1.20 | 2254 | 1.31 |
| HU6DL.A54 | 1573 | 4.23 | 4143 | 2.48 | 4590 | 1.88 | 2221 | 2.81 |
| HU6DL.I54 | 1933 | 4.66 | 4219 | 2.25 | 4542 | 2.42 | 2458 | 6.11 |

5. Conclusion of the experiment:

The above data show that each HU6DL mutant of the present disclosure has good affinity to NCI-N87, HCC827, SW780 and RT4 tumor cells.

**Example 8: HU6DL mutant-mediated TROP2 endocytosis experiment**

1. Objective of the experiment:

The antibody endocytic activity of HU6DL mutants was evaluated by flow cytometry, on tumor cell lines expressing TROP-2 antigen.

2. Experimental steps:

Gastric cancer cells NCI-N87 (purchased from Chinese Academy of Sciences Cell Bank, TCHu130) in a well-growing state were treated with Accutase (Sigma, cat: A6964) digestion solution. Single-cell suspension was prepared in 2% FBS (diluted in DBPS, pH 7.4) solution and the cell concentration was adjusted to $1 \times 10^7$ cells/mL. The suspension was aliquoted into a 96-well V-shaped bottom plate at 100 μL/well. The candidate antibody solution was added at a final concentration of 20 μg/mL, mixed well and incubated at 4°C for 1 h.

The plate was centrifuged at 300 g × 5 min at 4°C. Mouse-anti-human IgG Fc, PE-labeled secondary antibody (Biolegend, cat: 409304) solution diluted at a ratio of 5 μL/$10^6$ cells was added at 100 μL/well and incubated at 4°C for 1 h.

The plate was centrifuged at 300 g × 5 min at 4°C and washed twice. The cell pellet was resuspended in 1 mL of pre-warmed complete medium and aliquoted into four groups, namely the 0 min group, the blank group, the 30 min group and the 120 min group, respectively. The 0 min group and the blank control were placed on ice. The rest were placed in an incubator at 37°C for endocytosis for 30 min and 120 min, respectively. The group was taken out at the corresponding time point and pre-cooled on ice for 5 min. All treatment groups were centrifuged and the supernatant was discarded (4°C, 1500 rpm × 5 min). The cells were washed once with FACS buffer and the supernatant

was discarded. 250 μL of strip buffer was added to all treatment groups except the 0 min group, incubated at room temperature for 8 min and centrifuged (4°C, 1500 rpm × 5 min), and the supernatant was discarded. The cells were washed twice with FACS buffer and the supernatant was discarded. 80 μL of 2% FBS were added to each group of sample to resuspend the cells. The fluorescence signal of the sample to be tested was detected by a ZE5 flow cytometry (Bio-Rad, ZE5).

3. Data processing:

The endocytosis rate of each candidate antibody was calculated according to the following equation: antibody endocytosis percentage (%) = (MFI of treatment group - MFI of blank control group) / (MFI of 0 min group - MFI of blank control group) × 100%. The endocytosis rate of HU6DL mutants was detected by the above method and was as shown in Table 9:

Table 9. The endocytosis rate of HU6DL mutants by NCI-N87 cells

| Name | Antibody endocytosis (%) | |
| --- | --- | --- |
| | 30 min | 120 min |
| HU6DL.R54 | 64 | 62 |
| HU6DL.Y54 | 71 | 71 |
| HU6DL.Q54 | 71 | 71 |
| HU6DL.L54 | 76 | 72 |
| HU6DL.T54 | 63 | 69 |
| HU6DL.I54 | 65 | 66 |
| HU6DL.F54 | 72 | 75 |
| HU6DL.E54 | 75 | 72 |
| HU6DL.A54 | 72 | 70 |

4. Conclusion of the experiment:

The above data show that the HU6DL mutants of the present disclosure are well endocytosed in gastric cancer cells NCI-N87 mediated by the TROP-2 protein.

**Example 9: Detection of impurity components of HU6DL mutants**

1. Objective of the experiment: The content and change in level of the impurity peaks of the mutated antibodies were detected and compared, by capillary electrophoresis based on the Maurice-nrCE-SDS method.

2. Experimental steps

2.1. Sample preparation:

(1) Buffer exchange and concentration of samples: Buffer exchange and concentration of samples were required when the protein concentration of the sample was less than 5 mg/ml, or when the salt concentration in the sample buffer was high, so as to ensure a protein concentration of about 5 mg/ml and a salt concentration in the sample of less than 50 mM.

(2) Non-reducing processing of CE samples: The sample was added to an EP tube at an amount of 50 μg protein taken for each sample. 1 μl of 10 kD internal standard (Protein Simple, 046-144) was added, 2.5 μl of 250 mM IAM (Sigma, I1149-5G) was added, and 1 × sample buffer (Protein Simple, 046-567) was added to a final volume of 50 μl.

(3) The sample was mixed well by shaking, heated and incubated at 70°C for 10 min, and then taken out. The sample was incubated on ice for 5 min until cool and then centrifuged at 12,000 rpm for 5 min. After centrifugation, 35 μl of supernatant was transferred to a 96-well sample plate that matched the instrument, and then centrifuged at 1,000 rpm for 5 min. The 96-well sample plate was placed in Maurice (Protein Simple) for sample loading and analysis.

2.2. Detection on an instrument

The instrument was turned on and the software was opened. The instrument selfchecking was performed according to the instrument operation steps. The capillary cartridge was installed and the prepared corresponding reagents were put into the corresponding position in the instrument. The corresponding parameters were set and non-reducing CE analysis was performed according to the instrument operation steps. The sample sequence was set by editing the corresponding sequence according to the sample name, and the number of samples for each sequence would not exceed 48. After completion of sequence editing, detection of the sequence was started by clicking Start.

The content of the main peak and impurity peak of the sample was calculated using the following equation.

$$\text{Non-reducing purity (\%)} = \frac{\text{CA}_{\text{main peak}}}{\text{CA}_{\text{total}}} \times 100\%$$

Note:

In the equation, the non-reducing purity is the percentage of corrected main peak area;
$\text{CA}_{\text{main peak}}$ is the corrected main peak area;
$\text{CA}_{\text{total}}$ is the sum of the corrected main peak and impurity peak areas.

3. Results of the experiment:

Table 10. Detection of impurity components

| Sample | Main peak content (%) | Impurity peak content (%) |
|---|---|---|
| HU6DL.T54 | 93.62 | N/A |
| HU6DL | 84.78 | 4.72 |

The above data show that the main peak content of the sample HU6DL.T54 reached 93.62%, and no impurities were detected. The experimental results show that the $N^{54}D^{55}S^{56}$ motif in HCDR2 of HU6DL is susceptible to glycosylation, and site-directed mutation of N54 can effectively reduce glycosylated impurities and achieve better purity.

**Example 10: Competitive binding of humanized antibodies to antigen**

1. Objective of the experiment: The binding mode and binding site of different antibodies to antigen was studied by competitive binding experiments.
2. Experimental steps

The coating antibodies hRS7 and HU6DL mutants were diluted with pH 7.4 PBS to a concentration of 1 $\mu$g/ml, added to a 96-well high-affinity ELISA plate at 100 $\mu$l/well and incubated in a refrigerator at 4°C overnight (16-20 hours). After washing the plate 4 times with PBST (pH 7.4 PBS comprising 0.05% Tween-20), 2% bovine serum albumin (BSA) blocking solution diluted with PBST was added at 200 $\mu$l/well and incubated at room temperature for 1 hour for blocking. After completion of the blocking, the blocking solution was discarded, and the plate was washed 4 times with PBST buffer.

The competing antibodies hRS7 and HU6DL were diluted to 100 $\mu$g/ml with PBST comprising 0.5% BSA and added to the ELISA plate at 50 $\mu$l/well. TROP-2 His protein (Aero biosystems, cat# TR2-H5223) was diluted with PBST comprising 0.5% BSA at concentrations corresponding to affinity $2\text{xEC}_{80}$ or $2\text{xEC}_{90}$ of TROP-2 His protein to the coating antibody, respectively, and added to the ELISA plate at 50 $\mu$l/well. The ELISA plate was incubated at room temperature for 1 hour. After completion of the incubation, the plate was washed 4 times with PBST. Anti-His HRP labeled secondary antibody (Sino Biological, cat# 105327-MM02T-H) diluted with PBST comprising 0.5% BSA at 1:5000 was added at 100 $\mu$l/well and incubated at room temperature for 1 h. The plate was washed 4 times with PBST, then TMB chromogenic substrate (Cell Signaling Technology, cat#7004S) was added at 100 $\mu$l/well and incubated at room temperature in the dark for 1 minute. The stop solution (Cell Signaling Technology, cat#7002S) was added at 50 $\mu$l/well to terminate the reaction.

3. Data processing

The absorbance at 450 nm was read with a microplate reader (Thermo, model Varioskan LUX) and the data were analyzed, as shown in Table 11 below.

Table 11. Competitive binding of humanized antibodies and hRS7 to antigen

| Humanized antibody | Inhibition rate |
|---|---|
| hRS7 | 94.67% |
| HU6DL.T54 | 23.30% |

4. Results of the experiment

The mutationally modified humanized antibody of the present invention has a very low inhibition rate on the binding of hRS7 antibody to TROP2 protein, suggesting that the mutationally modified humanized antibody of the present invention and hRS7 antibody do not competitively bind the same epitope.

**Claims**

1. An anti-TROP-2 antibody, an antigen-binding fragment thereof or a mutant thereof, which comprises a heavy chain variable region and a light chain variable region, wherein,

the heavy chain variable region comprises HCDR1 as shown in SEQ ID NO: 4, HCDR2 as shown in SEQ ID NO: 43, and HCDR3 as shown in SEQ ID NO: 6,
the light chain variable region comprises LCDR1 as shown in SEQ ID NO: 7, LCDR2 as shown in SEQ ID NO: 8, and LCDR3 as shown in SEQ ID NO: 9;
preferably,
the heavy chain variable region comprises HCDR1 as shown in SEQ ID NO: 4, HCDR2 as shown in SEQ ID NO: 30, and HCDR3 as shown in SEQ ID NO: 6, the light chain variable region comprises LCDR1 as shown in SEQ ID NO: 7, LCDR2 as shown in SEQ ID NO: 8, and LCDR3 as shown in SEQ ID NO: 9; or,
the heavy chain variable region comprises HCDR1 as shown in SEQ ID NO: 4, HCDR2 as shown in SEQ ID NO: 26, and HCDR3 as shown in SEQ ID NO: 6, the light chain variable region comprises LCDR1 as shown in SEQ ID NO: 7, LCDR2 as shown in SEQ ID NO: 8, and LCDR3 as shown in SEQ ID NO: 9; or,
the heavy chain variable region comprises HCDR1 as shown in SEQ ID NO: 4, HCDR2 as shown in SEQ ID NO: 27, and HCDR3 as shown in SEQ ID NO: 6, the light chain variable region comprises LCDR1 as shown in SEQ ID NO: 7, LCDR2 as shown in SEQ ID NO: 8, and LCDR3 as shown in SEQ ID NO: 9; or,
the heavy chain variable region comprises HCDR1 as shown in SEQ ID NO: 4, HCDR2 as shown in SEQ ID NO: 28, and HCDR3 as shown in SEQ ID NO: 6, the light chain variable region comprises LCDR1 as shown in SEQ ID NO: 7, LCDR2 as shown in SEQ ID NO: 8, and LCDR3 as shown in SEQ ID NO: 9; or,
the heavy chain variable region comprises HCDR1 as shown in SEQ ID NO: 4, HCDR2 as shown in SEQ ID NO: 29, and HCDR3 as shown in SEQ ID NO: 6, the light chain variable region comprises LCDR1 as shown in SEQ ID NO: 7, LCDR2 as shown in SEQ ID NO: 8, and LCDR3 as shown in SEQ ID NO: 9; or,
the heavy chain variable region comprises HCDR1 as shown in SEQ ID NO: 4, HCDR2 as shown in SEQ ID NO: 39, and HCDR3 as shown in SEQ ID NO: 6, the light chain variable region comprises LCDR1 as shown in SEQ ID NO: 7, LCDR2 as shown in SEQ ID NO: 8, and LCDR3 as shown in SEQ ID NO: 9; or,
the heavy chain variable region comprises HCDR1 as shown in SEQ ID NO: 4, HCDR2 as shown in SEQ ID NO: 40, and HCDR3 as shown in SEQ ID NO: 6, the light chain variable region comprises LCDR1 as shown in SEQ ID NO: 7, LCDR2 as shown in SEQ ID NO: 8, and LCDR3 as shown in SEQ ID NO: 9; or,
the heavy chain variable region comprises HCDR1 as shown in SEQ ID NO: 4, HCDR2 as shown in SEQ ID NO: 41, and HCDR3 as shown in SEQ ID NO: 6, the light chain variable region comprises LCDR1 as shown in SEQ ID NO: 7, LCDR2 as shown in SEQ ID NO: 8, and LCDR3 as shown in SEQ ID NO: 9; or,
the heavy chain variable region comprises HCDR1 as shown in SEQ ID NO: 4, HCDR2 as shown in SEQ ID NO: 42, and HCDR3 as shown in SEQ ID NO: 6, the light chain variable region comprises LCDR1 as shown in SEQ ID NO: 7, LCDR2 as shown in SEQ ID NO: 8, and LCDR3 as shown in SEQ ID NO: 9.

2. The anti-TROP-2 antibody, antigen-binding fragment thereof or mutant thereof according to claim 1, wherein the antibody is a murine antibody, a chimeric antibody, a human antibody or a humanized antibody.

**3.** The anti-TROP-2 antibody, antigen-binding fragment thereof or mutant thereof according to claim 1 or 2, wherein:

the anti-TROP-2 antibody, antigen-binding fragment thereof or mutant thereof further comprises a constant region derived from human IgG1, IgG2, IgG3 or IgG4 or a mutant thereof;
preferably comprises a heavy chain constant region derived from a human IgG1, IgG2 or IgG4 mutant;
more preferably comprises an IgG1 heavy chain constant region introduced with 239D and 241L mutations;
further preferably comprises the heavy chain constant region of SEQ ID NO: 12.

**4.** The anti-TROP-2 antibody, antigen-binding fragment thereof or mutant thereof according to claim 3, wherein the anti-TROP-2 antibody, antigen-binding fragment thereof or mutant thereof further comprises a light chain constant region derived from human κ chain, λ chain or a mutant thereof; preferably comprises the light chain constant region of SEQ ID NO: 13.

**5.** The anti-TROP-2 antibody, antigen-binding fragment thereof or mutant thereof according to any one of claims 1 to 4, wherein the anti-TROP-2 antibody, antigen-binding fragment thereof or mutant thereof comprises a heavy chain variable region selected from the group consisting of SEQ ID NO: 20, SEQ ID NO: 16, SEQ ID NO: 17, SEQ ID NO: 18, SEQ ID NO: 19, SEQ ID NO: 31, SEQ ID NO: 32, SEQ ID NO: 33, SEQ ID NO: 34, or a heavy chain variable region having at least 70%, 75%, 80%, 85%, 90%, 95% or 99% identity with the same.

**6.** The anti-TROP-2 antibody, antigen-binding fragment thereof or mutant thereof according to any one of claims 1-2 and 5, wherein the anti-TROP-2 antibody, antigen-binding fragment thereof or mutant thereof comprises the light chain variable region of SEQ ID NO: 11, or a light chain variable region having at least 70%, 75%, 80%, 85%, 90%, 95% or 99% identity with the same.

**7.** The anti-TROP-2 antibody, antigen-binding fragment thereof or mutant thereof according to claim 5 or 6, wherein the anti-TROP-2 antibody or mutant thereof comprises a heavy chain selected from the group consisting of SEQ ID NO: 25, SEQ ID NO: 21, SEQ ID NO: 22, SEQ ID NO: 23, SEQ ID NO: 24, SEQ ID NO: 35, SEQ ID NO: 36, SEQ ID NO: 37, SEQ ID NO: 38, or a heavy chain having at least 80%, 85%, 90%, 95% or 99% identity with the same.

**8.** The anti-TROP-2 antibody, antigen-binding fragment thereof or mutant thereof according to claim 5 or 6, wherein the anti-TROP-2 antibody or mutant thereof comprises the light chain of SEQ ID NO: 15, or a light chain having at least 80%, 85%, 90%, 95% or 99% identity with the same.

**9.** The anti-TROP-2 antibody, antigen-binding fragment thereof or mutant thereof according to any one of claims 1 to 8, wherein:

the anti-TROP-2 antibody, antigen-binding fragment thereof or mutant thereof comprises the heavy chain variable region as shown in SEQ ID NO: 20, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or, the anti-TROP-2 antibody, antigen-binding fragment thereof or mutant thereof comprises the heavy chain variable region as shown in SEQ ID NO: 16, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or, the anti-TROP-2 antibody, antigen-binding fragment thereof or mutant thereof comprises the heavy chain variable region as shown in SEQ ID NO: 17, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or, the anti-TROP-2 antibody, antigen-binding fragment thereof or mutant thereof comprises the heavy chain variable region as shown in SEQ ID NO: 18, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or, the anti-TROP-2 antibody, antigen-binding fragment thereof or mutant thereof comprises the heavy chain variable region as shown in SEQ ID NO: 19, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or, the anti-TROP-2 antibody, antigen-binding fragment thereof or mutant thereof comprises the heavy chain variable region as shown in SEQ ID NO: 31, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or, the anti-TROP-2 antibody, antigen-binding fragment thereof or mutant thereof comprises the heavy chain variable region as shown in SEQ ID NO: 32, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or, the anti-TROP-2 antibody, antigen-binding fragment thereof or mutant thereof comprises the heavy chain variable region as shown in SEQ ID NO: 33, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same; or, the anti-TROP-2 antibody, antigen-binding fragment thereof or mutant thereof comprises the heavy chain variable region as shown in SEQ ID NO: 34, or a heavy chain variable region having at least 70%, 75%, 80%, 85%, 90%, 95% or 99% identity with the same, and the light chain variable region as shown in SEQ ID NO: 11, or a light chain variable region having at least 70%, 75%, 80%, 85%, 90%, 95% or 99% identity with the same.

10. The anti-TROP-2 antibody, antigen-binding fragment thereof or mutant thereof according to any one of claims 1 to 9, wherein:

the anti-TROP-2 antibody or mutant thereof comprises the heavy chain as shown in SEQ ID NO: 25, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or, the anti-TROP-2 antibody or mutant thereof comprises the heavy chain as shown in SEQ ID NO: 21, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or, the anti-TROP-2 antibody or mutant thereof comprises the heavy chain as shown in SEQ ID NO: 22, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or, the anti-TROP-2 antibody or mutant thereof comprises the heavy chain as shown in SEQ ID NO: 23, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or, the anti-TROP-2 antibody or mutant thereof comprises the heavy chain as shown in SEQ ID NO: 24, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or, the anti-TROP-2 antibody or mutant thereof comprises the heavy chain as shown in SEQ ID NO: 35, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or, the anti-TROP-2 antibody or mutant thereof comprises the heavy chain as shown in SEQ ID NO: 36, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or, the anti-TROP-2 antibody or mutant thereof comprises the heavy chain as shown in SEQ ID NO: 37, or a heavy chain having at least 80%, 85%, 90%, 95% or 99% identity with the same, and the light chain as shown in SEQ ID NO: 15, or a light chain having at least 80%, 85%, 90%, 95% or 99% identity with the same; or, the anti-TROP-2 antibody or mutant thereof comprises the heavy chain as shown in SEQ ID NO: 38, or a heavy chain having at least 80%, 85%, 90%, 95% or 99% identity with the same, and the light chain as shown in SEQ ID NO: 15, or a light chain having at least 80%, 85%, 90%, 95% or 99% identity with the same.

11. A polynucleotide encoding the anti-TROP-2 antibody, antigen-binding fragment thereof or mutant thereof according to any one of claims 1 to 10.

12. An expression vector comprising the polynucleotide according to claim 11.

13. A host cell transformed with the expression vector according to claim 12, wherein the host cell is selected from the group consisting of bacterium, yeast and mammalian cell, preferably *Escherichia coli, Pichia pastoris,* CHO cell or HEK293 cell.

14. A method of producing the anti-TROP-2 antibody, antigen-binding fragment thereof or mutant thereof according to any one of claims 1 to 10, the method comprises the following steps:

culturing the host cell according to claim 13, preferably HEK293 cells;
isolating the antibody from the culture, preferably isolating the antibody from the cell culture fluid; and
purifying the antibody, preferably purifying the antibody by chromatographic methods.

15. A pharmaceutical composition comprising the anti-TROP-2 antibody, antigen-binding fragment thereof or mutant thereof according to any one of claims 1 to 10, as well as a pharmaceutically acceptable excipient, diluent or carrier.

16. A detection or diagnostic reagent comprising the anti-TROP-2 antibody, antigen-binding fragment thereof or mutant thereof according to any one of claims 1 to 10.

17. Use of the anti-TROP-2 antibody, antigen-binding fragment thereof or mutant thereof according to any one of claims 1 to 10, or the pharmaceutical composition according to claim 15 in the preparation of a medicament, wherein the medicament is for treating or preventing a TROP-2-mediated disease or condition.

18. Use of the anti-TROP-2 antibody, antigen-binding fragment thereof or mutant thereof according to any one of claims 1 to 10, or the detection or diagnostic reagent according to claim 16 in the preparation of a kit, wherein the kit is for detecting, diagnosing or prognosing a TROP-2-mediated disease or condition.

19. The use according to claim 17 or 18, wherein the TROP-2-mediated disease or condition is a cancer;

preferably, the TROP-2-mediated disease or condition is a cancer expressing TROP-2;
said cancer is selected from the group consisting of breast cancer, non-small cell lung cancer, ovarian cancer, prostate cancer, pancreatic cancer, kidney cancer, lung cancer, liver cancer, gastric cancer, colon cancer, bladder cancer, esophageal cancer, cervical cancer, gallbladder cancer, glioblastoma and melanoma.

## INTERNATIONAL SEARCH REPORT

| International application No. |
|---|
| **PCT/CN2021/123731** |

### A.  CLASSIFICATION OF SUBJECT MATTER

C07K 16/30(2006.01)i;  A61K 39/395(2006.01)i;  A61P 35/00(2006.01)i

According to International Patent Classification (IPC) or to both national classification and IPC

### B.  FIELDS SEARCHED

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

TWABS; CNABS; VEN; CNTXT; WOTXT; USTXT; EPTXT; CNKI; 万方; ISI WEB OF KNOWLEDGE: applicant, inventor, 抗体, antibody, TROP-2, 滋养层细胞表面抗原2, 肿瘤相关的钙信号转导子2, TACSTD2, trophoblast cell surface antigen, 突变, mutation, 人源化, humanization, SEQ ID NOs: 4-43

### C.  DOCUMENTS CONSIDERED TO BE RELEVANT

| Category* | Citation of document, with indication, where appropriate, of the relevant passages | Relevant to claim No. |
|---|---|---|
| PX | CN 112243443 A (JIANGSU HANSOH PHARMACEUTICAL GROUP CO., LTD. et al.) 19 January 2021 (2021-01-19)<br>claims 1-25 | 1-19 |
| A | CN 107406508 A (ABRUZZO THERANOSTIC S.R.L.) 28 November 2017 (2017-11-28)<br>entire document | 1-19 |
| A | CN 111018992 A (BIO-THERA SOLUTIONS, LTD.) 17 April 2020 (2020-04-17)<br>entire document | 1-19 |
| A | CN 104053672 A (RINAT NEUROSCIENCE CORP.) 17 September 2014 (2014-09-17)<br>entire document | 1-19 |
| A | CN 110317822 A (YINGWEIFUSAI BIOTECHNOLOGY CO., LTD.) 11 October 2019 (2019-10-11)<br>entire document | 1-19 |
| A | CN 102827282 A (LIN, Hong) 19 December 2012 (2012-12-19)<br>entire document | 1-19 |
| A | US 2016313339 A1 (IMMUNOMEDICS, INC.) 27 October 2016 (2016-10-27)<br>entire document | 1-19 |

☑ Further documents are listed in the continuation of Box C.   ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **23 December 2021** | **17 January 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

# EP 4 230 656 A1

**INTERNATIONAL SEARCH REPORT**

| International application No. |
| --- |
| **PCT/CN2021/123731** |

**C.    DOCUMENTS CONSIDERED TO BE RELEVANT**

| Category* | Citation of document, with indication, where appropriate, of the relevant passages | Relevant to claim No. |
| --- | --- | --- |
| A | WO 2015126548 A1 (IBC PHARMACEUTICALS, INC.) 27 August 2015 (2015-08-27) <br> entire document | 1-19 |
| A | RAJI, R. et al. "Uterine and ovarian carcinosarcomas overexpressing Trop-2 are sensitive to hRS7, a humanized anti-Trop-2 antibody" <br> *JOURNAL OF EXPERIMENTAL AND CANCER RESEARCH,* <br> Vol. 30, No. 106, 10 November 2011 (2011-11-10), <br> pp. 1-7 | 1-19 |

Form PCT/ISA/210 (second sheet) (January 2015)

**INTERNATIONAL SEARCH REPORT**

| International application No. |
| --- |
| **PCT/CN2021/123731** |

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**
**Information on patent family members**

| International application No. |
| --- |
| **PCT/CN2021/123731** |

| Patent document cited in search report | | | Publication date (day/month/year) | Patent family member(s) | | | Publication date (day/month/year) |
| --- | --- | --- | --- | --- | --- | --- | --- |
| CN | 112243443 | A | 19 January 2021 | None | | | |
| CN | 107406508 | A | 28 November 2017 | AU | 2015357054 | A1 | 29 June 2017 |
| | | | | US | 10501555 | B2 | 10 December 2019 |
| | | | | EP | 3227340 | A1 | 11 October 2017 |
| | | | | CA | 2968330 | A1 | 09 June 2016 |
| | | | | JP | 2018500930 | A | 18 January 2018 |
| | | | | WO | 2016087651 | A1 | 09 June 2016 |
| | | | | EP | 3227340 | B1 | 17 June 2020 |
| | | | | US | 2018002437 | A1 | 04 January 2018 |
| CN | 111018992 | A | 17 April 2020 | AU | 2018267660 | C1 | 12 November 2020 |
| | | | | CN | 111087471 | A | 01 May 2020 |
| | | | | JP | 2020503243 | A | 30 January 2020 |
| | | | | EP | 3464381 | A1 | 10 April 2019 |
| | | | | AU | 2018267660 | A1 | 28 February 2019 |
| | | | | CN | 110526978 | A | 03 December 2019 |
| | | | | WO | 2019029715 | A1 | 14 February 2019 |
| | | | | AU | 2018267660 | B2 | 07 May 2020 |
| | | | | CN | 107446050 | A | 08 December 2017 |
| | | | | US | 2019048095 | A1 | 14 February 2019 |
| | | | | CN | 109078181 | A | 25 December 2018 |
| | | | | CN | 109078181 | B | 05 November 2019 |
| | | | | EP | 3464381 | A4 | 11 December 2019 |
| | | | | CA | 3038423 | A1 | 14 February 2019 |
| CN | 104053672 | A | 17 September 2014 | ZA | 201403492 | B | 26 April 2017 |
| | | | | TW | 201333036 | A | 16 August 2013 |
| | | | | US | 2016257746 | A1 | 08 September 2016 |
| | | | | EP | 2776470 | A2 | 17 September 2014 |
| | | | | RU | 2014116406 | A | 20 December 2015 |
| | | | | MX | 2014005728 | A | 30 May 2014 |
| | | | | AR | 088693 | A1 | 25 June 2014 |
| | | | | US | 10100114 | B2 | 16 October 2018 |
| | | | | JP | 5936702 | B2 | 22 June 2016 |
| | | | | CO | 7010785 | A2 | 31 July 2014 |
| | | | | AU | 2012335205 | A1 | 29 May 2014 |
| | | | | CA | 2954166 | A1 | 16 May 2013 |
| | | | | US | 2013122020 | A1 | 16 May 2013 |
| | | | | JP | 2014534233 | A | 18 December 2014 |
| | | | | US | 2014357844 | A1 | 04 December 2014 |
| | | | | TW | I495644 | B | 11 August 2015 |
| | | | | JP | 2016104751 | A | 09 June 2016 |
| | | | | CA | 2854720 | A1 | 16 May 2013 |
| | | | | SG | 11201401699 W | A | 26 September 2014 |
| | | | | US | 9399074 | B2 | 26 July 2016 |
| | | | | US | 2019002559 | A1 | 03 January 2019 |
| | | | | JP | 6157575 | B2 | 05 July 2017 |
| | | | | JP | 6475277 | B2 | 27 February 2019 |
| | | | | IL | 232426 | D0 | 30 June 2014 |
| | | | | WO | 2013068946 | A2 | 16 May 2013 |
| | | | | JP | 2017141249 | A | 17 August 2017 |
| | | | | US | 8871908 | B2 | 28 October 2014 |

Form PCT/ISA/210 (patent family annex) (January 2015)

**INTERNATIONAL SEARCH REPORT**
Information on patent family members

International application No.

**PCT/CN2021/123731**

| Patent document cited in search report | | | Publication date (day/month/year) | Patent family member(s) | | | Publication date (day/month/year) |
|---|---|---|---|---|---|---|---|
| | | | | CA | 2854720 | C | 18 December 2018 |
| | | | | BR | 112014011331 | A2 | 25 April 2017 |
| | | | | HK | 1201851 | A1 | 11 September 2015 |
| | | | | KR | 20140091040 | A | 18 July 2014 |
| | | | | WO | 2013068946 | A3 | 25 July 2013 |
| CN | 110317822 | A | 11 October 2019 | CN | 110317822 | B | 05 June 2020 |
| CN | 102827282 | A | 19 December 2012 | CN | 102827282 | B | 11 February 2015 |
| US | 2016313339 | A1 | 27 October 2016 | AU | 2016252771 | A1 | 10 August 2017 |
| | | | | US | 2018149657 | A1 | 31 May 2018 |
| | | | | US | 10436788 | B2 | 08 October 2019 |
| | | | | CN | 107428837 | A | 01 December 2017 |
| | | | | JP | 2018517888 | A | 05 July 2018 |
| | | | | JP | 6746845 | B2 | 26 August 2020 |
| | | | | US | 2019369103 | A1 | 05 December 2019 |
| | | | | WO | 2016172427 | A1 | 27 October 2016 |
| | | | | CA | 2981543 | A1 | 27 October 2016 |
| | | | | EP | 3286224 | A1 | 28 February 2018 |
| | | | | EP | 3286224 | A4 | 14 November 2018 |
| | | | | US | 9797907 | B2 | 24 October 2017 |
| WO | 2015126548 | A1 | 27 August 2015 | EP | 3107577 | A1 | 28 December 2016 |
| | | | | EP | 3107577 | A4 | 11 October 2017 |
| | | | | CN | 106132436 | A | 16 November 2016 |
| | | | | JP | 2017507936 | A | 23 March 2017 |
| | | | | JP | 6447933 | B2 | 09 January 2019 |
| | | | | AU | 2015219495 | A1 | 04 August 2016 |
| | | | | AU | 2015219495 | B2 | 21 November 2019 |
| | | | | MX | 2016010683 | A | 11 May 2017 |
| | | | | JP | 2019048869 | A | 28 March 2019 |
| | | | | JP | 6759508 | B2 | 23 September 2020 |
| | | | | CA | 2937236 | A1 | 27 August 2015 |

Form PCT/ISA/210 (patent family annex) (January 2015)

37

## REFERENCES CITED IN THE DESCRIPTION

**Patent documents cited in the description**

- WO 202011099107 A
- US 5840854 A
- US 6653104 B
- US 7420040 B
- CN 102827282 A
- CN 104114580 A

**Non-patent literature cited in the description**

- *J. Biol. Chem,* 1968, vol. 243, 3558
- **HOLLIGER ; HUDSON.** *Nat. Biotechnol.,* 2005, vol. 23, 1126-1136
- The Immunoglobulin FactsBook. 2001